# EUROPEAN PATENT APPLICATION

(11) **EP 4 527 393 A1**
(43) Date of publication of application: **26.03.2025**
(21) Application number: 23807565.9
(22) Date of filing: 12.05.2023
(51) Int. Cl.: A61K 33/22, A61K 9/14, A61K 41/00, A61K 47/04, A61K 47/60, A61K 47/64, A61K 47/69, A61P 35/00, C01B 32/28, C08G 65/06

(54) **SURFACE-MODIFIED NANODIAMOND**

(30) Priority: 20.05.2022 JP 2022083332
(71) Applicant: Kyoto University, Kyoto-shi, Kyoto 606-8501 (JP); Daicel Corporation, Osaka-shi, Osaka 530-0011 (JP)
(72) Inventor: NISHIKAWA, Masahiro, Tokyo 108-8230 (JP); KOMATSU, Naoki, Kyoto-shi, Kyoto 606-8501 (JP)
(74) Representative: Grünecker Patent- und Rechtsanwälte PartG mbB
(86) International application number: PCT/JP2023/017906
(87) International publication number: WO 2023/223962

(57) **Abstract**

To provide a surface-modified nanodiamond that enables selective accumulation of a sufficient amount of boron atoms in a cancer tissue or a cancer cell and has high killing and injuring ability against the cancer cell. The surface-modified nanodiamond of the present disclosure contains a nanodiamond particle, and a boron cluster-containing group and a group capable of recognizing a cancer cell as surface modification groups of the nanodiamond particle.

## Description

### Technical Field

The present disclosure relates to a surface-modified nanodiamond. The present disclosure claims priority to JP 2022-083332 filed in Japan on May 20, 2022, the content of which is incorporated herein by reference.

### Background Art

Boron neutron capture therapy (BNCT) is one type of advanced cancer therapy, in which cancer cells are destroyed (killed or injured) by alpha rays (⁴He nuclei) and lithium nuclei (⁷Li nuclei) emitted by the nuclear reaction between boron (¹⁰B) and neutrons. The distances that the α-ray and the ⁷Li nucleus generated by the nuclear reaction can travel are approximately several microns, respectively, and are approximately the same as the cell size. For this reason, if a sufficient amount of boron atoms (a boron compound) accumulates selectively in cancer tissues or cancer cells, cancer cells can be selectively killed or injured.

Currently, p-boronophenylalanine (BPA) is used in clinical applications as a boron drug used in BNCT (Patent Document 1). BPA is known to be taken up into a cancer cell via an L-amino acid transporter (LAT-1) expressed on the surface of the cancer cell. However, penetration of BPA through the cell membrane (cellular uptake and excretion) via LAT-1 is reversible, and BPA is rapidly metabolized (excreted) due to a low molecular weight compound. Thus, it is required to increase extracellular BPA concentration for maintaining the BPA concentration in a cancer cell during neutron irradiation. That is, continuous administration of high-concentration BPA is required in BNCT.

To overcome the above problem with BPA, nanoparticle-based drugs (nanodrugs), which can selectively deliver boron atoms into cancer cells and maintain the boron atoms therein, have been studied, and various boron-containing nanoparticles have been reported to date (Non-Patent Literature 1).

### Citation List

### Patent Document

Patent Document 1: JP 2009-51766 A

### Non-Patent Literature

Non-Patent Literature 1: M. Nishikawa et. al., Bull. Chem. Soc. Jpn. 2021, 94, 2302-2312.

### Summary of Invention

### Technical Problem

Although the drug described in Non-Patent Literature 1 exhibits a certain degree of significant effect as a drug in boron neutron capture therapy (BNCT), a higher effect is desired from the viewpoint of killing and injuring ability against a cancer cell.

Therefore, an object of the present disclosure is to provide a surface-modified nanodiamond that enables selective accumulation of a sufficient amount of boron atoms in a cancer tissue or a cancer cell and has high ability to kill and injure the cancer cell.

### Solution to Problem

As a result of intensive studies to achieve the above object, the inventors of the present disclosure have found that the above problem can be solved by a surface-modified nanodiamond containing a nanodiamond particle and, as surface modification groups of the nanodiamond particle, a boron cluster-containing group and a group capable of recognizing a cancer cell. The invention according to the present disclosure has been completed based on these findings.

That is, the present disclosure relates to a surface-modified nanodiamond containing:
a nanodiamond particle; and
a boron cluster-containing group and a group capable of recognizing a cancer cell as surface modification groups of the nanodiamond particle.

The surface-modified nanodiamond of the present disclosure preferably further contains a hydrophilic polymer chain-containing group as a surface modification group of the nanodiamond particle.

A hydrophilic polymer chain in the hydrophilic polymer chain-containing group is preferably a polyglycerol chain.

The boron cluster-containing group is preferably bonded to the nanodiamond particle via a hydrophilic polymer chain.

The group capable of recognizing a cancer cell is preferably bonded to the nanodiamond particle via a hydrophilic polymer chain.

The group capable of recognizing a cancer cell is preferably bonded to a hydrophilic polymer chain via an amide bond, an ester bond, or a thioester bond, and the hydrophilic polymer chain is preferably bonded to the nanodiamond particle.

The group capable of recognizing a cancer cell is preferably a group derived from a molecule capable of recognizing a cancer cell, and the molecule capable of recognizing a cancer cell is preferably a boronic acid, an amino acid, a peptide, a protein, folic acid, or a porphyrin.

A boron atom in the boron cluster-containing group is preferably enriched in ¹⁰B.

The present disclosure also describes a composition for use in boron neutron capture therapy, the composition containing the surface-modified nanodiamond.

### Advantageous Effects of Invention

The surface-modified nanodiamond of the present disclosure enables selective accumulation of a sufficient amount of boron atoms in a cancer tissue or a cancer cell and has high ability to kill and injure the cancer cell.

### Brief Description of Drawings

FIG. 1 is a diagram illustrating ¹³C-NMR measurement results of DND-PG in Example 1.
FIG. 2 is a diagram illustrating ¹H-NMR measurement results of DND-PG-COOH in Example 1.
FIG. 3 is a diagram illustrating ¹³C-NMR measurement results of DND-PG-COOH in Example 1.
FIG. 4 is a diagram illustrating an FT-IR spectrum of DND-PG(OTs)-COOH in Example 1.
FIG. 5 is a diagram illustrating a ¹H-NMR spectrum of DND-PG(BSH)-COOH in Example 1.
FIG. 6 is a diagram illustrating an FT-IR spectrum of DND-PG(BSH)-COOH in Example 1.
FIG. 7 is a diagram illustrating a ¹H-NMR spectrum of DND-PG(BSH)-PBA in Example 1.
FIG. 8 is a diagram illustrating (a) an FT-IR spectrum of DND-PG(OTs)-COOH, (b) an FT-IR spectrum of DND-PG(N₃)-COOH, (c) an FT-IR spectrum of DND-PG(¹⁰BSH)-COOH, (d) an FT-IR spectrum of DND-PG(¹⁰BSH)-PBA, and (e) an FT-IR spectrum of DND-PG(¹⁰BSH)-c(RGDyK) in Example 2.
FIG. 9 is a diagram illustrating (a) a ¹H-NMR spectrum, (b) a ¹³C-NMR spectrum, and (c) a ¹⁰B-NMR spectrum of DND-PG(¹⁰BSH)-COOH in Example 2.
FIG. 10 is a diagram illustrating (a) a ¹H-NMR spectrum and (b) a ¹⁰B-NMR spectrum of ¹⁰BSH(Pgy) in Example 2.
FIG. 11 is a diagram illustrating (a) a ¹H-NMR spectrum of DND-PG(¹⁰BSH)-PBA and (b) a ¹H-NMR spectrum of DND-PG(¹⁰BSH)-c(RGDyK) in Example 2.
FIG. 12 is a diagram illustrating results of a cytotoxicity test (cell viability of B16 mouse melanoma cells) using a nanoparticle of Example 2 (n = 6, Student's t-test with blank (PBS): *p < 0.5, **p < 0.01, ***p < 0.001).
FIG. 13 is a graph illustrating the cell viability of B16 cells exposed to the nanoparticle of Example 2 with respect to irradiation dose in a colony formation assay when thermal neutron irradiation was performed.
FIG. 14 is a graph illustrating the cell viability of B16 cells exposed to the nanoparticle of Example 2 with respect to irradiation dose in a colony formation assay when thermal neutron irradiation was performed (significance of slope difference *p < 0.5, **p < 0.01, ***p < 0.001).
FIG. 15 is a diagram illustrating TEM images in a cell introduction experiment of a) control, b) DND-PG(¹⁰BSH)-PBA, and c) DND-PG(¹⁰BSH)-c(RGDyK) in Example 2.
FIG. 16 is a diagram illustrating TEM images in a cell introduction experiment of d) DND-PG-COOH, e) DND-PG-PBA, and f) DND-PG-c(RGDyK) in Example 2.

### Description of Embodiments

### Surface-modified nanodiamond

The surface-modified nanodiamond (hereinafter, the nanodiamond may be referred to as "ND") according to an embodiment of the present disclosure contains a nanodiamond particle; and a boron cluster-containing group and a group capable of recognizing a cancer cell as surface modification groups that modify the surface of the nanodiamond particle. The surface-modified nanodiamond of the present disclosure preferably further contains a hydrophilic polymer chain-containing group as a surface modification group of the nanodiamond particle. The surface-modified ND according to an embodiment of the present disclosure may have only one type of the surface modification groups described above or may have two or more types of the surface modification groups described above.

The boron cluster-containing group and the group capable of recognizing a cancer cell may be present in the same surface modification group or different surface modification groups. When the surface-modified nanodiamond according to an embodiment of the present disclosure contains a hydrophilic polymer chain-containing group as the surface modification group, the boron cluster-containing group, the group capable of recognizing a cancer cell, and the hydrophilic polymer chain-containing group may be present in the same surface modification group or may be present in different surface modification groups. That is, the surface modification group may contain two or three of the boron cluster-containing group, the group capable of recognizing a cancer cell, and the hydrophilic polymer chain-containing group all together, or may contain only one of these groups.

The surface-modified ND according to an embodiment of the present disclosure contains the boron cluster-containing group, and thus tends to have improved killing and injuring ability against a cancer cell. The reason for this is considered as follows: because a cancer cell is killed or injured by alpha particles or lithium nuclei generated by the nuclear reaction between neutrons and boron, the surface-modified ND according to an embodiment of the present disclosure has improved killing and injuring ability against the cancer cell, since the surface-modified ND contains the boron cluster-containing group, and thus the boron atom concentration is high in the surface-modified ND. The boron cluster-containing group may have a property of accumulating the surface-modified ND according to an embodiment of the present disclosure in or around a cancer tissue and a cancer cell in a non-specific manner. Examples of such a boron cluster include a carborane described below. However, the boron cluster-containing group and the group capable of recognizing a cancer cell are independent from each other. The group capable of recognizing a cancer cell has a property of selectively recognizing a cancer tissue and/or a cancer cell, preferably specifically recognizing a specific type of cancer cell from a number of cells, accumulating the surface-modified ND according to an embodiment of the present disclosure in or around the cancer tissue and/or the cancer cell, and further improving the boron atom-derived killing and injuring ability against a cancer cell. The surface-modified ND according to an embodiment of the present disclosure tends to have higher killing and injuring ability against a cancer cell than that of a surface-modified ND having only a group containing a boron atom and being capable of recognizing a cancer cell (for example, a group derived from an aromatic boronic acid such as phenylboronic acid). The reason for this is considered as follows: since the surface-modified ND according to an embodiment of the present disclosure has the boron cluster-containing group and the group capable of recognizing a cancer cell independently as described above, the killing and injuring ability against the cancer cell is improved by a synergistic effect of the groups. When the surface-modified ND according to an embodiment of the present disclosure contains a hydrophilic polymer chain-containing group, the surface-modified ND tends to have more excellent stability under physiological conditions. Furthermore, the surface-modified ND tends to easily migrate to cancer tissues and cancer cells.

The surface modification group in the surface-modified ND according to an embodiment of the present disclosure may have the boron cluster-containing group together with the hydrophilic polymer chain-containing group. For example, the boron cluster-containing group may be bonded to the ND particle via a hydrophilic polymer chain. When the surface modification group is in the form as described above, the surface-modified ND according to an embodiment of the present disclosure tends to have further improved killing and injuring ability against a cancer cell, and have more excellent stability under physiological conditions.

The surface modification group in the surface-modified ND according to an embodiment of the present disclosure may have the group capable of recognizing a cancer cell together with the hydrophilic polymer chain-containing group. For example, the group capable of recognizing a cancer cell may be bonded to the ND particle via a hydrophilic polymer chain. The group capable of recognizing a cancer cell may be bonded to a hydrophilic polymer chain via an amide bond, an ester bond, or a thioester bond, and the hydrophilic polymer chain may be bonded to the ND particle. When the surface modification group is in the form as described above, a sufficient amount of boron atoms can be selectively accumulated in a cancer tissue or a cancer cell, and the surface-modified ND tends to have more excellent stability under physiological conditions.

The surface modification group in the surface-modified ND according to an embodiment of the present disclosure may have the boron cluster-containing group, the group capable of recognizing a cancer cell, and the hydrophilic polymer chain-containing group all together. For example, the boron cluster-containing group and the group capable of recognizing a cancer cell may be bonded to the ND particle via the same hydrophilic polymer chain.

The ND particle constituting the surface-modified ND according to an embodiment of the present disclosure preferably contains a nanodiamond primary particle. The ND particle may contain a secondary particle formed by aggregation (adhesion) of a plurality of the primary particles. The surface-modified ND according to an embodiment of the present disclosure may also have, on the surface of the ND particle, one type or two or more types of substituents besides the boron cluster-containing group, the group capable of recognizing a cancer cell, and the hydrophilic polymer chain-containing group. Examples of such a substituent include an amino group, a hydroxy group, and a carboxyl group.

The ND particle in the surface-modified nanodiamond according to an embodiment of the present disclosure is not particularly limited, and examples of usable ND particles include a detonation ND, an ND produced by a high temperature-high pressure method, and an ND produced by a chemical vapor deposition method (an ND obtained by pulverizing a diamond thin film prepared by a CVD method). Of these, the detonation ND is preferred from the viewpoint of even better dispersibility in a dispersion medium and a primary particle size of a single-digit nanometer. The above-described ND particle may have, in the diamond crystal structure thereof, a defect with fluorescent properties or magnetic properties, such as a nitrogen-vacancy center (NV center) or a silicon-vacancy center (SiV center).

### Boron cluster-containing group

In the surface-modified ND according to an embodiment of the present disclosure, the boron cluster-containing group can be formed by introducing a boron cluster into the ND particle. The ND particle may be an ND particle containing a hydrophilic polymer chain-containing group as a surface modification group. In this case, the boron cluster-containing group may be directly bonded to the surface of the ND particle or may be bonded to the ND particle via a hydrophilic polymer chain.

### Boron cluster

The boron cluster is a cluster having a boron atom in its molecule. The boron cluster is not particularly limited, and examples thereof include a carborane, a dodecaborate, a boron ion cluster having a molecular formula of B₁₀H₁₀, and a sandwich-type ion cluster having a molecular formula of M(C₂B₉H₁₀)₂ (M is a transition element). The carborane may be a nido-carborane (ortho form, meta form, para form) having a molecular formula of C₂B₉H₁₁ or a closo-carborane having a molecular formula of CB₁₁H₁₂. The dodecaborate (BH) is an ion cluster having a molecular formula of B ₁₂H₁₂. The transition element M constituting the sandwich-type ion cluster having a molecular formula of M(C₂B₉H₁₀)₂ may be Fe, Co, Ni, Mo, or the like. Such a boron cluster may have a substituent such as a thiol group, a hydroxy group, an amino group, or a carboxy group.

The boron atom in the boron cluster may be any isotope such as ¹⁰B or ¹¹B, but the boron atom is preferably enriched in ¹⁰B.

In the surface-modified ND according to an embodiment of the present disclosure, when a boron cluster is introduced into an ND particle or an ND particle containing a hydrophilic polymer chain-containing group as a surface modification group, the boron cluster may be bonded to the ND particle or the hydrophilic polymer chain as the surface modification group via a single bond or a linking group. Examples of the linking group include an alkenyl group, a sulfide bond, a sulfonium bond, a sulfoxide group, a secondary amine group, a tertiary amine group, a quaternary amine group (quaternary ammonium cationic group), an amide bond, an ether bond, an ester bond, a thioester bond, a heterocyclic ring (for example, 1,2,3-triazole ring), a carbonyl group, an acetal bond, and a group formed by bonding of two or more of these groups. Of these, the linking group is preferably a group containing a sulfide bond, a group containing a 1,2,3-triazole ring, or a group containing a sulfide bond and a 1,2,3-triazole ring.

In the surface-modified ND according to an embodiment of the present disclosure, when a boron cluster is introduced into an ND particle in which at least one of CH₂OH groups of a polyglycerol chain described below is substituted with a carboxy group, the boron cluster is preferably introduced into the ND particle by, for example, (1) a reaction between a substituent of the boron cluster, such as a thiol group, a hydroxy group, or an amino group, and a hydroxy group of the polyglycerol chain, or (2) a reaction (that is, a click reaction or the like) between an alkynyl group of the boron cluster and a polyglycerol chain in which some of its hydroxy groups are substituted with an azide group, from the viewpoint of reactivity. In the case (1), in the surface-modified ND according to an embodiment of the present disclosure, the boron cluster is bonded to the polyglycerol chain via a linking group such as a sulfide bond or an ether bond. In the case (2), in the surface-modified ND according to an embodiment of the present disclosure, the boron cluster is bonded to the polyglycerol chain via a 1,2,3-triazole ring (preferably a sulfide bond and a 1,2,3-triazole ring).

### Group capable of recognizing cancer cell

In the surface-modified ND according to an embodiment of the present disclosure, the group capable of recognizing a cancer cell can be formed by introducing a molecule capable of recognizing a cancer cell into the ND particle. The ND particle may be an ND particle containing a hydrophilic polymer chain-containing group as a surface modification group. In this case, the group capable of recognizing a cancer cell may be directly bonded to the surface of the ND particle or may be bonded to the ND particle via a hydrophilic polymer chain.

### Molecule capable of recognizing cancer cell

Examples of the molecule capable of recognizing a cancer cell include a boronic acid, an amino acid, a peptide, a protein, folic acid, and a porphyrin. For example, a boronic acid-derived group can be formed by introducing the boronic acid into the ND particle. In addition, a peptide-derived group or a protein-derived group can be formed by introducing the peptide or the protein into the ND particle.

The boronic acid is not particularly limited, and examples thereof include compounds represented by Formula (A) below.

R^{a2}-B(-R^{a1})₂ (A)

In Formula (A), R^{a1} represents a hydroxy group which may be protected by a protective group. R^{a2} represents a hydrogen atom or a monovalent organic group.

Examples of the protective group that may be possessed by the hydroxy group of R^{a1} include C₁₋₄ alkyl groups, such as a methyl group, an ethyl group, and a t-butyl group; a group that forms an acetal bond together with an oxygen atom constituting the hydroxy group (e.g., a C₁₋₄ alkyl-O-C₁₋₄ alkyl group, such as a methoxymethyl group); a group that forms an ester bond together with the oxygen atom constituting the hydroxy group (e.g., an acetyl group or a benzoyl group).

Examples of the monovalent organic group of R^{a2} include a substituted or unsubstituted hydrocarbon group (monovalent hydrocarbon group, in particular, a monovalent aliphatic or aromatic hydrocarbon group), a substituted or unsubstituted heterocyclic group (monovalent heterocyclic group), and a group formed by bonding of two or more of the monovalent hydrocarbon group and/or the monovalent heterocyclic group. From the viewpoint of ease of production and stability under physiological conditions, the monovalent organic group is preferably a substituted or unsubstituted monovalent aromatic hydrocarbon group or an aromatic heterocycle-containing group (i.e., the heterocyclic group having aromaticity).

Examples of the aromatic hydrocarbon group include an aromatic hydrocarbon group having from 6 to 18 (preferably from 6 to 14) carbons. Examples of the aromatic heterocycle-containing group include an aromatic heterocycle-containing group having from 4 to 18 (preferably from 6 to 14) carbons and from 1 to 4 heteroatoms of at least one type selected from a nitrogen atom, an oxygen atom, and a sulfur atom.

The boronic acid is not particularly limited, but is preferably an aromatic boronic acid, and more preferably a phenylboronic acid such as 3-aminophenylboronic acid, 4-aminophenylboronic acid, or 4-aminomethylphenylboronic acid, or a pyridineboronic acid such as (2-aminopyridin-4-yl)boronic acid, (5-aminopyridin-3-yl)boronic acid, (2-aminopyridin-5-yl)boronic acid or (4-aminopyridin-2-yl)boronic acid.

The amino acid is not particularly limited, and examples thereof include leucine, valine, isoleucine, phenylalanine, tyrosine, lysine, arginine, histidine, and derivatives thereof. Such an amino acid may be introduced into the ND particle (for example, a COOH group of DND-PG-COOH) via an amino group or a carboxy group contained in the molecule.

The peptide is not particularly limited, and examples thereof include a peptide having an RGD sequence. The RGD sequence means Arg-Gly-Asp (arginine-glycine-aspartic acid). Such an RGD sequence is not particularly limited, and examples thereof include GRGDNP (Gly-Arg-Gly-Asp-Asn-Pro), Cyclo(-Arg-Gly-Asp-D-Phe-Cys), Cyclo[-Arg-Gly-Asp-D-Phe-Lys (Cys)], Cyclo(-Arg-Gly-Asp-D-Tyr-Lys), and Cyclo(-Arg-Gly-Asp-D-Phe-Lys). Of these, Cyclo(-Arg-Gly-Asp-D-Tyr-Lys) is preferred. When such a peptide is introduced into the ND particle, a peptide having Lys as a free amino group is preferred from the viewpoint that an amide bond can be formed by its ε-amino group. Alternatively, the peptide may be introduced into the ND particle (particularly, a COOH group of DND-PG-COOH) from the terminal COOH of the peptide via or not via a spacer.

The protein is not particularly limited, but is preferably, for example, an IgG antibody targeting a cancer cell (in particular, a receptor or a sugar chain on a cancer cell surface). Specific examples of the protein include an anti-EGFR antibody (anti-epidermal growth factor receptor antibody, "Erbitux" (cetuximab)).

In the surface-modified ND according to an embodiment of the present disclosure, when a molecule capable of recognizing a cancer cell is introduced into an ND particle or an ND particle containing a hydrophilic polymer chain-containing group as a surface modification group, the molecule capable of recognizing a cancer cell may be introduced directly or via a substituent of the molecule. That is, the molecule capable of recognizing a cancer cell may be bonded to the ND particle or the hydrophilic polymer chain as the surface modification group via a single bond or a linking group, and the linking group is preferably a group derived from a substituent of the molecule capable of recognizing a cancer cell, for example, a sulfide bond, a sulfonium bond, a sulfoxide group, a secondary amine group, a tertiary amine group, a quaternary amine group (a quaternary ammonium cationic group), an amide bond, an ether bond, an ester bond, a thioester bond, a carbonyl group, or an acetal bond. Of these, an amide bond is more preferred.

In the surface-modified ND according to an embodiment of the present disclosure, when a molecule capable of recognizing a cancer cell is introduced into an ND particle in which at least one of CH₂OH groups of a polyglycerol chain described below is substituted with a carboxy group, the molecule capable of recognizing a cancer cell is preferably introduced via a substituent such as a thiol group, a hydroxy group, or an amino group from the viewpoint of reactivity. In this case, in the surface-modified ND according to an embodiment of the present disclosure, the group capable of recognizing a cancer cell is bonded to the polyglycerol chain via an amide bond, an ester bond, or a thioester bond, and the hydrophilic polymer chain is bonded to the nanodiamond particle.

### Hydrophilic polymer chain-containing group

Examples of the hydrophilic polymer chain include a polymer chain containing a structural unit derived from a monomer containing a hydrophilic group, such as a polyether chain (e.g., poly(ethylene oxide), poly(propylene oxide), or a copolymer of these), or a polyglycerol chain (e.g., C₃H₆O(CH₂CH(OH)CH₂O)n-H). Of these, the hydrophilic polymer chain is preferably a polyglycerol chain from the viewpoint of stability under physiological conditions. That is, the hydrophilic polymer chain-containing group is preferably a group containing a polyglycerol chain.

The polyglycerol chain is preferably a polyglycerol chain represented by Formula (1) below.

-(X¹C₃H₅)-(OC₃H₅)p-(X²R¹)q (1)

[In Formula (1), p represents an integer of 1 or more; and q represents an integer satisfying q = p + 2; X¹ represents a divalent group; a dangling bond, which extends leftward from X¹ in [X¹C₃H₅], is bonded to the ND particle; [X²R¹] represents a terminal of the polyglycerol chain, X² represents a single bond or a divalent group; and R¹ represents a hydrogen atom or a monovalent organic group.]

[X¹C₃H₅] in Formula (1) above is represented by [-X¹-CH₂-C(-)H-CH₂-]. X¹ in [X¹C₃H₅] above is bonded to the ND particle, and two C atoms are each bonded to O in [OC₃H₅] or X² in [X²R¹]. X¹ in [X¹C₃H₅] above represents a divalent group. One or more R¹s are a monovalent organic group, and the one or more monovalent organic groups of R¹s preferably contain the boron cluster-containing group or the group capable of recognizing a cancer cell.

Examples of the divalent group of X¹ include an amino group (-NR^{a}-), an amide bond (-NR^{a-}C(=O)-), an ether bond (-O-), an ester bond (-O-C(=O)-), a thioester bond (-S-C(=O)-), a phosphinate group (-PH(=O)O-), a phosphonate group (-P(-OH)(=O)O-), a phosphate ester (-O-P(=O)(OH)-O-), a sulfide bond (-S-), a carbonyl group (-C(=O)-), a urethane bond (-R^{a}N-C(=O)-O-), an imide bond (-C(=O)-NR^{a}-C(=O)-), a thiocarbonyl group (-C(=S)-), a siloxane bond (-Si-O-), a sulfate ester group (-O-S(=O)₂-O-), a sulfonyl group (-S(=O)₂-O-), a sulfone group (-S(=O)₂-), a sulfoxide group (-S(=O)-), and a group formed by bonding of two or more of these. In an asymmetric divalent group, the direction of the divalent group with respect to the ND particle side and the R side is not particularly limited. R^{a} above represents a hydrogen atom or a monovalent organic group. Among the above-described divalent groups, preferred is -NR^{a}-, -O-, -C(=O)O-, -NR^{a-}C(=O)-, -PH(=O)O-, or -S-, and more preferred is -NR^{a}-, - O-, -S-, -NR^{a-}C(=O)-, or -C(=O)O-.

Examples of the monovalent organic group of R¹ include a substituted or unsubstituted hydrocarbon group (monovalent hydrocarbon group, in particular, a monovalent aliphatic or aromatic hydrocarbon group), a substituted or unsubstituted heterocyclic group (monovalent heterocyclic group), a group formed by bonding of two or more of the monovalent hydrocarbon group and/or the monovalent heterocyclic group, the boron cluster-containing group, and the group capable of recognizing a cancer cell. The group formed by the bonding may be a group formed by direct bonding or bonding via a linking group. Examples of the linking group include an amino group, an ether bond, an ester bond, a phosphinate group, a sulfide bond, a carbonyl group, an organic group-substituted amide group, an organic group-substituted urethane bond, an organic group-substituted imide bond, a thiocarbonyl group, a siloxane bond, and a group formed by bonding of two or more of these.

[OC₃H₅] with p in Formula (1) above is a structure derived from a glycerol represented by [-O-CH₂-C(-)H-CH₂-] and forms a polyglycerol chain together with [X¹C₃H₅]. p represents the number of repeating units of [OC₃H₅] and is an integer of 1 or more, preferably from 3 to 2000, more preferably from 5 to 500, and even more preferably from 10 to 200. p may be the same or different in the group containing a plurality of polyglycerol chains.

In Formula (1), [X²R¹] represents a terminal of the polyglycerol chain and is bonded to C in [X¹C₃H₅] or C in [OC₃H₅].

X² in [X²R¹] above represents a single bond or a divalent group. Examples of the divalent group include the divalent groups exemplified and described as X¹ in [X¹C₃H₅] described above. R^{a} and R¹ in X² may be bonded to each other to form a ring. Among these, X² is preferably -NR^{a}-, -NR^{a}C(=O)-, -O-, -C(=O)O-, -O-C(=O)-, -S-C(=O)-, -C(=O)-, -PH(=O)O-, -O-P(=O)(OH)-O-, -S-, -O-S(=O)₂-O-, or -O-S(=O)₂-, and more preferably -NR^{a}-, -O-, -NR^{a}C(=O)-, -O-C(=O)-, -S-C(=O)-, or -C(=O)-. X¹ in [X¹C₃H₅] above and X² in [X²R¹] above may be the same as or different from each other. The plurality of [X²R¹]s may be the same or different. X² in [X²R¹] above may be the same or different in the group containing a plurality of polyglycerol chains. q represents an integer of 3 or more, and the value of q depends on the value of p and satisfies q = p + 2. q may be the same as or different from each other in the groups containing a plurality of polyglycerol chains.

In the case where R¹ above is a monovalent organic group, a plurality of R¹s in Formula (1) may be the same as or different from each other. Examples of the monovalent organic group include those exemplified and described as the monovalent organic group in R^{a} above. Specifically, examples of the monovalent organic group include a substituted or unsubstituted hydrocarbon group (monovalent hydrocarbon group), a substituted or unsubstituted heterocyclic group (monovalent heterocyclic group), and a group formed by bonding of two or more of these groups. The monovalent organic group may have an ionic form.

The group formed by bonding may be a group formed by direct bonding or bonding via a linking group. The hydrocarbon group in the substituted or unsubstituted hydrocarbon group is preferably an alkyl group, more preferably an alkyl group having from 1 to 18 carbons, still more preferably an alkyl group having from 1 to 6 carbons, and particularly preferably an ethyl group, a propyl group, a butyl group, or a hexyl group.

Specific examples of [X²R¹] include OH, NH₂, CH₃, an alkoxy group, an acyl group, a mono- or di-alkylamino group, a mono- or di-alkenylamino group, an alkylamide group, an alkenylamide group, a quaternary ammonium-substituted alkoxy group, a chlorine-substituted alkoxy group, a polyalkylene oxide group, a cyclic imide group, a carboxyl-substituted alkylamino group, a carboxyl-substituted alkyloxy group; the boron cluster-containing group, the group having a carbonyl group, an amide bond, an ester bond, a thioester bond, a sulfide bond, an ether bond, a heterocyclic ring (for example, a 1,2,3-triazole ring), and a group formed by bonding of two or more types thereof; and the group capable of recognizing a cancer cell, the group having a carbonyl group, an amide bond, an ester bond, or a thioester bond. Two or more [X²R¹]s above may form a ring via R¹. Of these, a cyclic imide group or a carboxyl-substituted alkylamino group is preferred from the viewpoint of more excellent water dispersibility of the surface-modified ND.

The number-average degree of polymerization of glycerol in the polyglycerol chain is preferably from 3 to 2000, more preferably from 5 to 500, and even more preferably from 10 to 200. When the number-average degree of polymerization is large, the repulsive force between nanodiamonds acts sufficiently, and the dispersibility of the ND particle can be further improved. When the number-average degree of polymerization is 5000 or less, the polyglycerol chains are less likely to be entangled between nanodiamonds, and the dispersibility of the ND particle in water can be further improved. The number-average degree of polymerization is defined as the number of glycidol units constituting a polyglycerol chain in a group bonded to one surface functional group of the raw material nanodiamond. The number of surface functional groups of the raw material nanodiamond may be determined by elemental analysis or acid value measurement of the raw material nanodiamond, or by these two techniques in combination.

The polyglycerol chain may be a polyglycerol chain in which some or all of hydroxy groups in the polyglycerol chain are substituted with an amino group (hereinafter referred to as "nitrogen-substituted polyglycerol chain"). Examples of the nitrogen-substituted polyglycerol chain include a polyglycerol chain represented by Formula (1) above in which at least a part of X²s is an amino group.

At least a part of the amino groups in the nitrogen-substituted polyglycerol chain may be protected by a compound containing an acidic functional group for the purpose of improving the dispersibility of the surface-modified ND in blood (preventing aggregation in blood). Examples of such a polyglycerol chain include a polyglycerol chain represented by Formula (1) above in which at least a part of X² is an amino group (-NR^{a}-) and at least a part of R^{a} is a protective group. Examples of the acidic functional group include a carboxy group and a sulfonyl group. Specific examples of the compound containing the acidic functional group include dicarboxylic acids, such as oxalic acid, malonic acid, succinic acid, glutaric acid, and adipic acid; acid anhydrides, such as oxalic anhydride, succinic anhydride, maleic anhydride, phthalic anhydride, and benzoic anhydride; and compounds containing a sulfonyl group, such as sulfuric acid. Of these, an acid anhydride is preferred from the viewpoint of forming an amide bond and having a carboxyl group at the terminal, and further improving the dispersibility of the surface-modified ND in blood. Examples of other protective groups include alkyl groups, such as a methyl group, an ethyl group, and a propyl group; and cycloalkyl groups, such as a benzyl group and a phenyl group.

The surface-modified ND preferably contains, as the surface modification group, a boronic acid-derived group as the group capable of recognizing a cancer cell. In this case, the boronic acid-derived group is preferably bonded to the ND particle via the hydrophilic polymer chain. The boron atom derived from the boronic acid is more preferably bonded to the hydrophilic polymer chain via an aromatic ring. The boron atom may be bonded to the aromatic ring directly or via another linking group, and is preferably bonded to the aromatic ring directly, particularly preferably bonded to a carbon atom constituting the aromatic ring. The surface-modified ND may have only one type of aromatic ring described above or may have two or more types of aromatic rings described above.

Examples of the aromatic ring include an aromatic hydrocarbon ring and an aromatic heterocycle. Examples of the hydrocarbon ring include C₆₋₁₄ hydrocarbon rings (particularly C₆₋₁₀ hydrocarbon rings), such as a benzene ring and a naphthalene ring. Examples of the aromatic heterocycle include rings having a carbon atom and at least one type of heteroatom (e.g., an oxygen atom, a sulfur atom, or a nitrogen atom) in atoms constituting the ring, and fused rings of these.

The aromatic ring is preferably a four- to ten-membered ring, more preferably a six-membered ring. Examples of the 6-membered aromatic ring include a benzene ring and a pyridine ring.

The aromatic ring may or may not have a substituent. Examples of the substituent include a halogen atom such as a fluorine atom, a chlorine atom, a bromine atom, or an iodine atom; a hydroxy group; a thiol group; a carboxy group; an amino group; and an oxo group. Of these, the aromatic ring preferably has an electron-withdrawing group such as a fluorine atom as the substituent. The electron-withdrawing group is preferably present at a meta position with respect to the bonding position of the boron atom in the aromatic ring. The substituent corresponds to R³ in Formula (2) described below.

The aromatic ring is preferably bonded to the hydrophilic polymer chain via a divalent hydrocarbon group, a sulfide bond, a sulfonium bond, a sulfoxide bond, a secondary amine group, a tertiary amine group, a quaternary amine group (quaternary ammonium cationic group), an amide bond, an ether bond, an ester bond, a thioester bond, a carbonyl group, an acetal bond, or a group formed by linking of a plurality of these.

Examples of the divalent hydrocarbon group include a methylene group, an ethylene group, a propylene group, and a trimethylene group. From the viewpoint of improving the dispersibility of the surface-modified ND, the divalent hydrocarbon group preferably has from 1 to 6 carbons, and more preferably from 1 to 3 carbons.

Next will be described an embodiment in which the boron atom is directly bonded to a carbon atom constituting the aromatic ring and the aromatic ring is bonded to the hydrophilic polymer chain via a linking group bonded to a carbon atom constituting the aromatic ring with reference to a group represented by Formula (2) below.

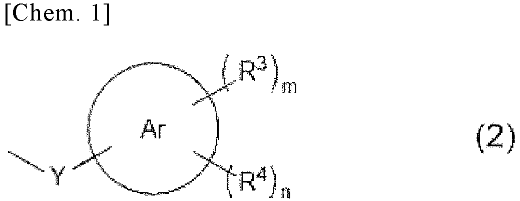

In Formula (2), Ar represents an aromatic ring, and R³ represents a substituent that may be possessed by the aromatic ring. The substituent is preferably, for example, an electron-withdrawing group. m is an integer of 0 or more. R⁴ is a group containing a boron atom derived from a boronic acid, and the boron atom is directly bonded to a carbon atom constituting the aromatic ring. n is an integer of 1 or more. Y is a divalent hydrocarbon group, a sulfide bond, a sulfonium bond, a sulfoxide group, a secondary amine group, a tertiary amine group, a quaternary amine group (quaternary ammonium cationic group), an amide bond, an ether bond, an ester bond, a thioester bond, a carbonyl group, an acetal bond, or a group formed by linking of a plurality of these. The dangling bond extending leftward from Y is bonded to a hydrophilic polymer chain (preferably a polyglycerol chain).

The boron atom in R⁴ may be bonded to a hydrogen atom, a hydroxy group, an amino group, a sulfo group, a monovalent organic group, or another substituent. The form containing the boron atom is not limited, and may have, for example, a group represented by Formula (3) below.

-B(-R⁵)₂ (3)

In Formula (3), R⁵s are identical to or different from each other and represent a hydrogen atom, a hydroxy group, an amino group, a sulfo group, or a monovalent organic group. In addition, two R⁵s may be bonded to each other to form a ring (hydrocarbon-containing ring). When two R⁵s are bonded to each other to form a ring, the ring may be formed via an oxygen atom. Furthermore, a carbon atom constituting the ring may form a double bond with an adjacent carbon atom. The number of carbons constituting the ring is not particularly limited and is, for example, preferably from 4 to 12, more preferably from 4 to 10, and even more preferably from 4 to 6.

The monovalent organic group is, for example, a hydrocarbon group, and is preferably an alkyl group, more preferably an alkyl group having from 1 to 6 carbons, even more preferably an alkyl group having from 1 to 3 carbons, and particularly preferably a methyl group.

Specific examples of the group represented by Formula (3) include boronic acid (-B(OH)₂) and borane (-BH₂). Of these, boronic acid is preferred.

The content of the boron atom derived from the boron cluster in the surface-modified ND according to an embodiment of the present disclosure is not particularly limited and is, for example, preferably 1.0 mass% or more, more preferably 5.0 mass% or more, and even more preferably 10 mass% or more. When the boron atom content is within the above range, there is a tendency that a sufficient amount of boron atoms can be selectively accumulated in a cancer tissue or a cancer cell. The upper limit of the boron atom content is not particularly limited, and is, for example, 40.0 mass%.

When the group capable of recognizing a cancer cell is a boronic acid-derived group in the surface-modified ND according to an embodiment of the present disclosure, the content of the boron atom derived from the boronic acid-derived group is not particularly limited and is, for example, preferably from 0.1 to 2.0 mass%, more preferably from 0.2 to 1.5 mass%, and even more preferably from 0.3 to 1.0 mass%. When the boron atom content is within the above range, the surface-modified ND tends to have excellent capability to recognize a cancer cell.

The boron atom may be any isotope, such as ¹⁰B and ¹¹B, and is not particularly limited. Of these, the boron atom is preferably enriched in ¹⁰B. Of these, the boron atom derived from the boron cluster is particularly preferably enriched in ¹⁰B.

In the surface-modified ND according to an embodiment of the present disclosure, the mass ratio of the ND to the surface modification group [ND/surface modification group of the present disclosure] is not particularly limited and is preferably from 0.1 to 2.0, more preferably from 0.2 to 1.5, and even more preferably from 0.2 to 1.0. In particular, when the mass ratio is 0.2 or more, the properties of a nanodiamond material are less likely to be impaired, and, when the mass ratio is 1.5 or less (furthermore, 1.0 or less), the degree of modification of the surface modification group becomes sufficient, and dispersibility under physiological conditions is more excellent. The mass ratio is determined as follows. Based on the weight loss rate measured by a thermogravimetric analysis of a sample before introduction of a boron substituent, the weight of the remaining ND is determined, and the lost weight is defined as the mass of the polyglycerol chain-containing surface modification group before introduction of the boron substituent. The mass ratio corresponds to the ratio of the weight of the ND to the sum of the mass of the surface modification group and the weight of the boron substituent to be introduced herein.

The volume average particle size (MV) of the surface-modified ND according to an embodiment of the present disclosure is, for example, 200 nm or less, preferably 100 nm or less, and more preferably 60 nm or less. The lower limit of the average particle size (MV) of the surface-modified ND is, for example, 5 nm. The average particle size of the surface-modified ND can be measured using a dynamic light scattering method.

The surface-modified ND according to an embodiment of the present disclosure can be used for boron neutron capture therapy of a tumor disease.

Examples of the "tumor disease" in the present specification include malignant melanoma, renal cancer, prostate cancer, breast cancer, lung cancer, pancreatic cancer, bowel cancer, hepatocellular carcinoma, biliary tract cancer, gastric cancer, ovarian cancer, esophageal cancer, urothelial carcinoma, colon cancer, bone cancer, skin cancer (e.g., malignant skin cancer), head and neck cancer, rectal cancer, cancer of the anal region, testicular cancer, uterine cancer, fallopian tube cancer, endometrial cancer, cervical cancer, vaginal cancer, small intestinal cancer, endocrine system cancer, thyroid cancer, parathyroid cancer, adrenal cancer, soft tissue sarcoma, urethral cancer, penile cancer, pediatric solid cancer, bladder cancer, malignant pleural mesothelioma, brain tumor (e.g., malignant brain tumor), and central nervous system tumor.

Boron neutron capture therapy using the surface-modified ND is performed by, for example, administering a drug containing the surface-modified ND to a mammal (e.g., a human) suffering from a tumor disease by any suitable route of administration that accumulates the compound at the target site. Preferably, the surface-modified ND is selectively accumulated in a tumor. A formulation containing the compound may be administered all at once or sequentially. Administration of the formulation may be repeated as necessary.

After the surface-modified ND reaches a site in the tumor, the site is irradiated with an effective amount of a low-energy neutron beam, such as a thermal neutron beam or an epithermal neutron beam. The site may be irradiated through the skin, or the site may be fully or partially exposed before irradiation and then irradiated. The administration of the surface-modified ND and the subsequent irradiation with the thermal neutron beam or epithermal neutron beam may be repeated as necessary.

The route of administration of the surface-modified ND is preferably intravenous administration, intraarterial administration, intramuscular administration, subcutaneous administration, intradermal administration, intraspinal administration, or intraperitoneal administration.

The surface-modified ND can be administered to a mammal suffering from a tumor disease within 72 hours (preferably from 5 minutes to 48 hours and more preferably from 30 minutes to 30 hours) before irradiation with the thermal neutron beam or the epithermal neutron beam. Alternatively, the surface-modified ND may be administered during irradiation with the thermal neutron beam or the epithermal neutron beam. A typical dose of the surface-modified ND is preferably in a range from 0.01 mg to 5000 mg/kg of body weight per irradiation with the thermal neutron beam or the epithermal neutron beam. The boron-10 (¹⁰B) dosage is preferably in a range from 0.005 mg to 1000 mg/kg of body weight per irradiation with the thermal neutron beam or the epithermal neutron beam. The irradiation time per irradiation with the thermal neutron beam or the epithermal neutron beam is preferably from 1 minute to 5 hours and more preferably from 10 minutes to 2 hours. The irradiation amount of the thermal neutron beam or the epithermal neutron beam is not particularly limited and may be an irradiation amount commonly used in neutron capture therapy.

The boron neutron capture therapy using the surface-modified ND may be applied as a single treatment or may be applied in combination with known surgery or chemotherapy. If desired, after removal of a tumor to a surgically possible extent, the remaining tumor can be destroyed by the boron neutron capture therapy using the surface-modified ND.

A composition containing the surface-modified ND can be used for the boron neutron capture therapy. The surface-modified ND may be used as is as an active ingredient in the composition, or the composition may be formulated by adding a pharmaceutically acceptable additive or the like. As described above, the composition may contain another boron compound, such as BPA or BSH.

Examples of the additive include, but are not limited to, purified water, saline, a phosphate buffer, dextrose, pharmaceutically acceptable organic solvents such as glycerol and ethanol, animal and vegetable oils, glucose, mannose, fructose, galactose, sorbitol, mannitol, crystalline cellulose, hydroxypropyl cellulose, starch, corn starch, silicic anhydride, magnesium aluminum silicate, collagen, poly(vinyl alcohol), polyvinylpyrrolidone, carboxyvinyl polymers, sodium carboxymethyl cellulose, sodium polyacrylate, sodium alginate, water-soluble dextran, sodium carboxymethyl starch, pectin, methylcellulose, ethylcellulose, xanthan gum, gum arabic, tragacanth, casein, agar, polyethylene glycol, diglycerol, glycerol, propylene glycol, vaseline, paraffin, octyldodecyl myristate, isopropyl myristate, higher alcohols, stearyl alcohol, stearic acid, and human serum albumin.

The surface-modified ND may be formulated with a pharmaceutically acceptable solvent, and a pharmaceutically acceptable carrier, such as an excipient, a binder, a stabilizer, or a dispersant, into a parenteral dosage form, such as a solution for injection, a suspension, an emulsion, a cream, an ointment, an inhalant, or a suppository. The surface-modified ND is preferably formulated into a solution for injection.

The solution for injection can be produced as a solution of the surface-modified ND in a pharmaceutically acceptable solvent. Such a solution may also contain a stabilizing component and/or a buffering component. The solution for injection may be a dry formulation to be used by adding a suitable solvent thereto before use.

### Method for producing surface-modified nanodiamond

The surface-modified ND according to an embodiment of the present disclosure can be produced by reacting an ND particle, a boron cluster, and a molecule capable of recognizing a cancer cell. Preferably, the surface-modified ND according to an embodiment of the present disclosure is produced by reacting an ND particle, a hydrophilic polymer, a boron cluster, and a molecule capable of recognizing a cancer cell. The reactions of the hydrophilic polymer, the boron cluster, and the molecule capable of recognizing a cancer cell with the ND particle may be performed simultaneously or separately. From the viewpoint of easily producing a desired surface-modified ND, it is preferable to react the ND particle with the hydrophilic polymer and then react the resulting reaction product with the boron cluster and the molecule capable of recognizing a cancer cell. The order in which the boron cluster and the molecule capable of recognizing a cancer cell are reacted with the reaction product of the ND particles and the hydrophilic polymer is not particularly limited.

The hydrophilic polymer is not particularly limited as long as the polymer contains a structural unit derived from a monomer having a hydrophilic group. Examples of the polymer include polyethers (such as poly(ethylene oxide), poly(propylene oxide), and copolymers of these) and polyglycerols (such as C₃H₆O(CH₂CH(OH)CH₂O)n-H). The polyglycerol may be a polyglycerol in which some or all of hydroxy groups are substituted with an amino group.

The method of reacting the ND particle with the hydrophilic polymer is not particularly limited, and the reaction can be performed by a known and commonly used method. A specific example of using a polyglycerol as the hydrophilic polymer will be described below.

The method for modifying the surface of the ND particle with a polyglycerol is not particularly limited. For example, the surface-modified ND particle can be produced by ring-opening polymerization of glycidol on the ND particle. The ND particle naturally has a carboxy group, a hydroxy group, or the like on the particle surface in the production process, and the ND particle surface can be modified with a polyglycerol chain by reacting such a functional group with glycidol.

The amount of glycidol to be used is, for example, from 0.5 to 100 parts by weight and preferably from 10 to 50 parts by weight per part by weight of the ND particle. The adjustment of the amount of glycidol to be used can control the chain length of the polyglycerol group and the bond density to the surface of the ND particle.

The ring-opening polymerization reaction can be carried out, for example, by heating a liquid mixture of a solvent and an ND particle and sequentially adding glycidol to the mixture. The heating temperature is, for example, from 40 to 180°C, preferably from 60 to 150°C, and particularly preferably from 80 to 125°C.

The solvent is not particularly limited, but is preferably a solvent having low reactivity with glycidol and high solubility for polyglycerol. The solvent is preferably an alcohol and particularly preferably an aliphatic alcohol. Examples of the aliphatic alcohol include aliphatic monohydric alcohols having from 2 to 4 carbons, such as ethanol, propanol, isopropyl alcohol, n-butanol, isobutyl alcohol, sec-butyl alcohol, and tert-butyl alcohol; and aliphatic polyhydric alcohols having from 2 to 4 carbons, such as ethylene glycol, propylene glycol, 1,3-propanediol, 1,3-butanediol, 1,4-butanediol, and glycerol. One type of these may be used alone, or two or more types thereof may be used in combination.

A catalyst may be used for the ring-opening polymerization reaction. The catalyst includes an acidic catalyst and a basic catalyst. Examples of the acidic catalyst include boron trifluoride etherate, acetic acid, and phosphoric acid. Examples of the basic catalyst include triethylamine, pyridine, dimethylaminopyridine, and triphenylphosphine.

The ring-opening polymerization reaction can be stopped by cooling the reaction liquid (e.g., by a method of adding water to the reaction system). The product can be purified by a known or commonly used method (e.g., water washing, centrifugation, or membrane filtration). This purification operation can remove the solvent, excess glycidol, and by-products (e.g., polyglycerol in a free state without binding to an ND particle). Thus, the ND particle having a group containing a polyglycerol chain is produced in the form of an aqueous dispersion.

In the ND particle having a surface modified with a polyglycerol chain, at least one of CH₂OH groups of the polyglycerol chain may be oxidized in the presence of an N-oxoammonium ion and a hypochlorite salt and/or a chlorite salt and substituted with a carboxy group.

When at least one of CH₂OH groups of the polyglycerol chain of the ND particle is substituted with a carboxy group, a boron cluster or a molecule capable of recognizing a cancer cell can be easily introduced into the polyglycerol chain, since the carboxy group has higher reactivity than the CH₂OH group.

In this case, the method preferably includes a step of oxidizing, with an N-oxoammonium ion, at least one of CH₂OH groups of the polyglycerol chain of the ND particle having a group containing the polyglycerol chain to convert the group into an aldehyde group or an equivalent thereof (a step of conversion into an aldehyde group or the like), and a step of oxidizing the aldehyde group or equivalent thereof to convert it into a carboxy group (a step of conversion into a carboxy group).

In the step of conversion into an aldehyde group or the like, at least one of CH₂OH groups contained in the polyglycerol chain of the ND particle having a group containing the polyglycerol chain is oxidized with an N-oxoammonium ion and converted into an aldehyde group or an equivalent thereof (e.g., a - CH(OH)₂ group).

The N-oxoammonium ion is an activator that promotes the oxidation reaction and has an action of oxidizing the CH₂OH group to convert it into an aldehyde group or an equivalent thereof.

The N-oxoammonium ion is produced, for example, by oxidizing a nitroxy radical compound with a hypochlorite salt.

The reaction of oxidizing the nitroxy radical compound with a hypochlorite salt is preferably carried out in the presence of an alkali metal bromide, such as sodium bromide, from the viewpoint of improving the reaction rate.

The amount of the alkali metal bromide to be used is, for example, from 1 to 40 times by mole and preferably from 10 to 20 times by mole the amount of the nitroxy radical compound.

Examples of the nitroxy radical compound include compounds represented by Formulae (c-1) to (c-3) below or precursors of these compounds. One type of these may be used alone, or two or more types thereof may be used in combination.

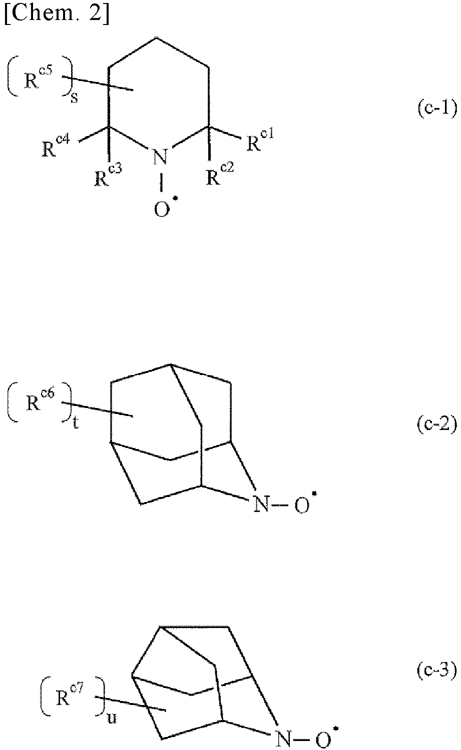

In Formula (c-1), R^{c1} to R^{c4} are identical or different and represent an alkyl group, R^{c5} represents a hydroxy group, a carboxy group, an acyl group, a substituted or unsubstituted amino group, an amide group, an alkoxy group, an aryloxy group, or an aralkyloxy group. s represents an integer of 0 to 4.

In Formula (c-2), R^{c6} represents an alkyl group, a substituted or unsubstituted amino group, an amide group, a halogen atom, or a hydroxy group. t represents an integer of 0 to 4.

In Formula (c-3), R^{c7} represents an alkyl group, a halogen atom, or a hydroxy group. u represents an integer of 0 to 4.

Examples of the alkyl group include alkyl groups having from 1 to 4 carbons, such as a methyl group and an ethyl group.

Examples of the acyl group include C₁₋₄ aliphatic acyl groups, such as a formyl group, an acetyl group, a propionyl group, a butyryl group, and an isobutyryl group; an acetoacetyl group; and C₇₋₁₄ aromatic acyl groups, such as a benzoyl group.

The substituted amino group includes a monosubstituted amino group and a disubstituted amino group. Examples of the substituent possessed by the substituted amino group include C₁₋₅ alkyl groups, C₃₋₆ cycloalkyl groups, C₆₋₁₀ aryl groups, and an acyl group. Examples of the acyl group include the same examples as those for the acyl group described above. Examples of the substituted amino group include an acetylamino group.

The amide group is a group represented by -NHCOR', and R' represents a hydrocarbon group. Examples of the hydrocarbon group include an alkyl group having from 1 to 4 carbons, a haloalkyl group having from 1 to 4 carbons, an aryl group having from 6 to 14 carbons, and an aralkyl group having from 4 to 15 carbons. The amide group includes an acetamide group and a benzamide group.

Examples of the haloalkyl group include C₁₋₄ haloalkyl groups, such as trifluoromethyl and trichloromethyl.

Examples of the alkoxy group include alkoxy groups having from 1 to 4 carbons, such as a methoxy group.

Examples of the aryloxy group include C₆₋₁₀ aryloxy groups, such as a phenoxy group.

Examples of the aralkyloxy group include C₇₋₁₆ aralkyloxy groups, such as a benzoyl group.

s, t, and u represent the number of groups shown in parentheses and are identical to or different from each other and integers of 0 to 4. In the case where s, t, and u are integers of 2 or more, two or more of the groups may be identical to or different from each other.

Examples of the compound represented by Formula (c-1) include 2,2,6,6-tetramethylpiperidine-1-oxyl represented by Formula (c-1-1) below, 4-hydroxy-2,2,6,6-tetramethylpiperidine-1-oxyl, 4-methoxy-2,2,6,6-tetramethylpiperidine-1-oxyl, 4-benzoyloxy-2,2,6,6-tetramethylpiperidine-1-oxyl, 4-amino-2,2,6,6-tetramethylpiperidine-1-oxyl, 4-acetamido-2,2,6,6-tetramethylpiperidine-1-oxyl, and 4-carboxy-2,2,6,6-tetramethylpiperidine-1-oxyl.

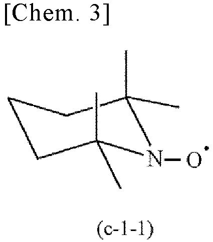

Examples of the compound represented by Formula (c-2) include 2-azaadamantane-N-oxyl represented by Formula (c-2-1) below, 1-methyl-2-azaadamantane-N-oxyl represented by Formula (c-2-2) below, 1,5-dimethyl-2-azaadamantane-N-oxyl represented by Formula (c-2-3) below, 5-amino-2-azaadamantane-N-oxyl, and 5-acetylamino-2-azaadamantane-N-oxyl.

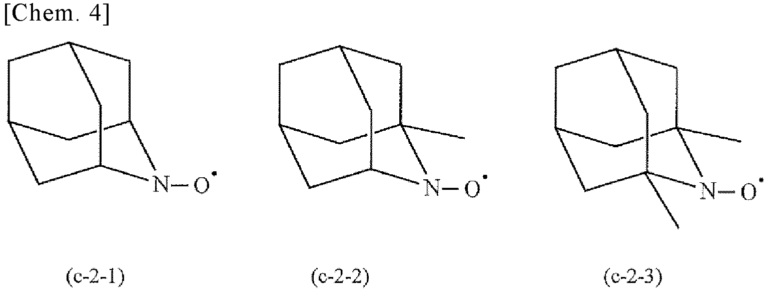

Examples of a precursor of the compound represented by Formula (c-2) include 2-hydroxy-2-azaadamantane represented by Formula (c-2-1') below.

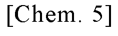

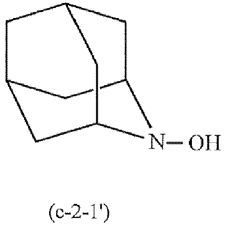

Examples of the compound represented by Formula (c-3) include 9-azanoradamantane-N-oxyl represented by Formula (c-3-1) below and 1,5-dimethyl-9-azanoradamantane-N-oxyl represented by Formula (c-3-2) below.

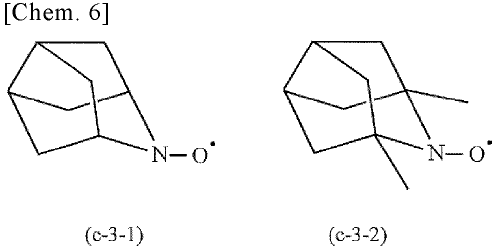

The amount of hypochlorous acid to be used is, for example, from 5 to 50 mol, preferably from 10 to 30 mol, and particularly preferably from 20 to 30 mol per mol of the nitroxy radical compound.

The nitroxy radical compound may be used as is, or may be used after being supported on a carrier.

The amount of the nitroxy radical compound to be used is, for example, from 0.005 to 1 part by weight and preferably from 0.01 to 0.5 parts by weight per part by weight of the surface modification group of the ND particle having a polyglycerol group.

The N-oxoammonium ion produced by oxidizing the nitroxy radical compound, where a CH₂OH group of the polyglycerol chain was oxidized, and the N-oxoammonium ion itself was reduced to N-hydroxylamine, has lost its oxidizing power. N-hydroxylamine is converted again into the N-oxoammonium ion (active form) by oxidation with a co-oxidant, and the N-oxoammonium ion can be reutilized in the oxidation reaction.

Examples of the co-oxidant include hypochlorite salts (i.e., salts of hypochlorous acid and an alkali metal or an alkaline earth metal), such as sodium hypochlorite, potassium hypochlorite, and calcium hypochlorite; chlorite salts (i.e., salts of chlorous acid and an alkali metal or an alkaline earth metal), such as sodium chlorite, potassium chlorite, and calcium chlorite; hydrogen peroxide, t-butyl hydroperoxide, trichloroisocyanuric acid, peracetic acid, performic acid, trichloroperacetic acid, trifluoroperacetic acid, and iodobenzene diacetate.

Among these co-oxidants, a hypochlorite salt, such as sodium hypochlorite, potassium hypochlorite, or calcium hypochlorite, is preferably used from the viewpoint of enabling efficient reactivation of N-hydroxylamine.

In the step of conversion into a carboxy group, the aldehyde group or equivalent thereof formed in the step of conversion into an aldehyde group or the like is oxidized and converted into a carboxy group.

In the step of conversion into a carboxy group, an oxidant can be used for the reaction. Examples of the oxidant include hypochlorite salts (i.e., salts of hypochlorous acid and an alkali metal or an alkaline earth metal), such as sodium hypochlorite, potassium hypochlorite, and calcium hypochlorite; and chlorite salts (i.e., salts of chlorous acid and an alkali metal or an alkaline earth metal), such as sodium chlorite, potassium chlorite, and calcium chlorite. One type of these may be used alone, or two or more types thereof may be used in combination.

In the case of using a hypochlorite salt as the co-oxidant in the step of conversion into an aldehyde group or the like and using a hypochlorite salt as the oxidant in the step of conversion into a carboxy group (Case 1), a series of reactions for converting a CH₂OH group contained in the polyglycerol chain into a carboxy group (a CH₂OH group → an aldehyde group or an equivalent thereof → a carboxy group) can be efficiently carried out in one pot.

In Case 1, the amount of the hypochlorite salt to be used is, for example, from 5 to 50 mol, preferably from 10 to 30 mol, and particularly preferably from 20 to 30 mol per mol of an N-oxoammonium ion.

In Case 1, the pH of the reaction system is desirably adjusted to fall within a range such that a hypochlorite salt is stably present and the oxidation capacity can be well exhibited, and, for example, the pH is preferably adjusted to fall within a range of 8 to 11.

In the case of using a hypochlorite salt as the co-oxidant in the step of conversion into an aldehyde group or the like and using a chlorite salt as the oxidant in the step of conversion into a carboxy group (Case 2), the amount of a carboxy group contained in the surface-modified ND particle can be controlled by adjusting the amount of the chlorite salt to be used and the reaction time. For example, a decrease in the amount of the chlorite salt to be used enables selective production of a surface-modified ND particle carboxylated to a small extent.

In Case 2, i.e., in the case of using a chlorite salt in the step of conversion into a carboxy group, when the chlorite salt oxidizes an aldehyde group or an equivalent thereof and converts it into a carboxy group, the chlorite salt itself is reduced to a hypochlorite salt. The thus-produced hypochlorite salt can be utilized as the co-oxidant in the step of conversion into an aldehyde group or the like.

In Case 2, the amount of the hypochlorite salt to be used is, for example, from 0.1 to 10 mol, preferably from 0.3 to 3 mol, and particularly preferably from 0.5 to 1.5 mol per mol of an N-oxoammonium ion.

In Case 2, the amount of the chlorite salt to be used is, for example, from 3 to 100 mol, preferably from 4 to 75 mol, and particularly preferably from 5 to 50 mol per mol of an N-oxoammonium ion.

In Case 2, the ratio of the chlorite salt to the hypochlorite salt (the former/the latter; molar ratio) is, for example, from 2 to 100, preferably from 3 to 75, and particularly preferably from 5 to 50.

In Case 2, the total amount of the hypochlorite salt and chlorite salt to be used is, for example, from 5 to 100 mol, preferably from 5 to 75 mol, and particularly preferably from 5 to 50 mol per mol of an N-oxoammonium ion.

In Case 2, the reaction can be carried out under mild conditions, and the pH of the reaction system is, for example, from 3 to 11 and preferably from 4 to 9.

An example of the reaction in Case 2 is shown in the following scheme.

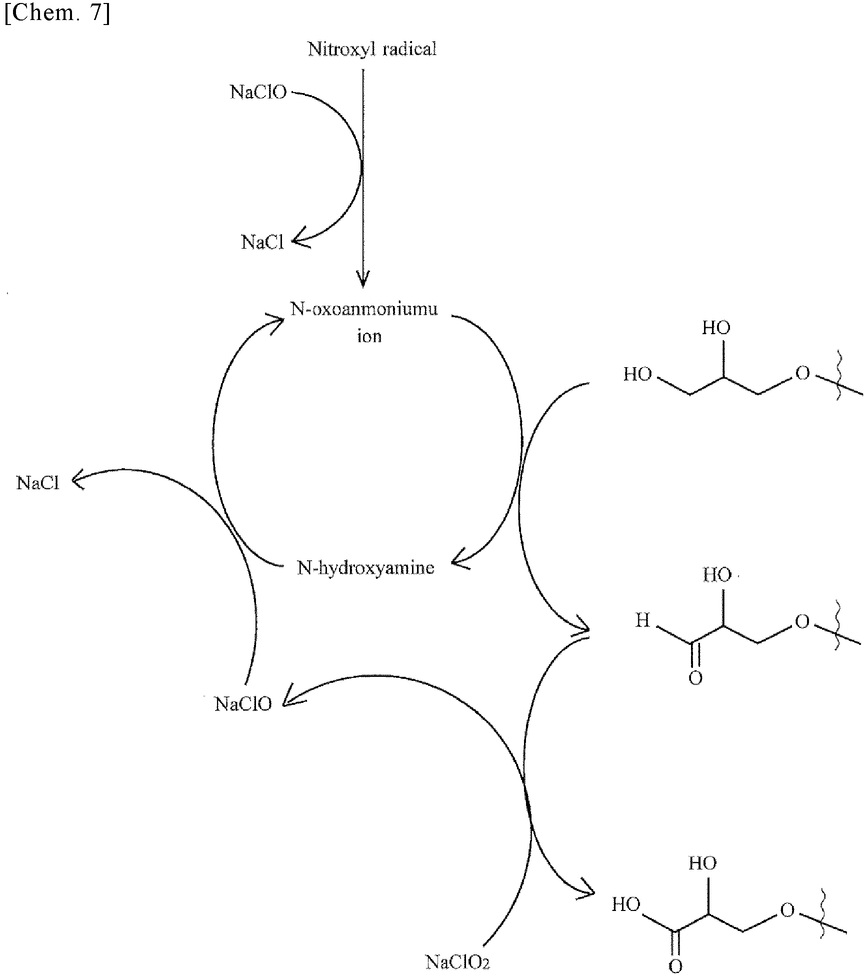

The method of reacting the ND particle with the boron cluster is not particularly limited, and the reaction can be performed by a known and commonly used method. A boron cluster-containing group can be introduced by reacting a functional group, such as a carboxy group or a hydroxy group, present on the surface of the ND particle, with a boron cluster in the same manner as in the case of reacting polyglycerol serving as the hydrophilic polymer with the ND particle.

When the surface-modified ND according to an embodiment of the present disclosure is produced by a method in which an ND particle is reacted with a hydrophilic polymer and then the resulting reaction product is reacted with a boron cluster, the boron cluster may be reacted with a hydrophilic polymer chain in the reaction product or may be reacted with a functional group present on the surface of the ND particle. In the case of the former reaction, the surface-modified ND according to an embodiment of the present disclosure contains a boron cluster-containing group via the hydrophilic polymer chain. In the case of the latter reaction, the surface-modified ND according to an embodiment of the present disclosure contains a boron cluster-containing group as the surface modification group on the surface of the ND particle.

The method of reacting the ND particle with the molecule capable of recognizing a cancer cell is not particularly limited, and the reaction can be performed by a known and commonly used method. A group capable of recognizing a cancer cell can be introduced by reacting a functional group, such as a carboxy group or a hydroxy group, present on the surface of the ND particle, with a molecule capable of recognizing a cancer cell in the same manner as in the case of reacting polyglycerol serving as the hydrophilic polymer with the ND particle.

When the surface-modified ND according to an embodiment of the present disclosure is produced by a method in which an ND particle is reacted with a hydrophilic polymer and then the resulting reaction product is reacted with a molecule capable of recognizing a cancer cell, the molecule capable of recognizing a cancer cell may be reacted with a hydrophilic polymer chain in the reaction product or may be reacted with a functional group present on the surface of the ND particle. In the case of the former reaction, the surface-modified ND according to an embodiment of the present disclosure contains a group capable of recognizing a cancer cell via the hydrophilic polymer chain. In the case of the latter reaction, the surface-modified ND according to an embodiment of the present disclosure contains a group capable of recognizing a cancer cell as the surface modification group on the surface of the ND particle.

The production method for the ND particle before surface modification is not particularly limited. Examples of usable NDs include a detonation ND (i.e., an ND produced by a detonation method, which may be referred to as "DND"), a high temperature-high pressure ND (i.e., an ND produced by a high temperature-high pressure method), and an ND produced by a chemical vapor deposition method (an ND produced by pulverizing a diamond thin film prepared by a CVD method). Of these, a detonation ND is preferred from the viewpoint of even better dispersibility in a dispersion medium and a primary particle size of a single-digit nanometer.

The detonation ND may be, for example, an ND produced through the formation step, acid treatment step, oxidation treatment step, drying step, surface functional group adjustment step, and disintegration-classification treatment step described below.

The formation step involves charging an explosive into a pressure-resistant container for detonation, sealing the container in a state where the explosive coexists with a gas having an atmospheric composition, and detonating the explosive in the container. For example, a mixture of TNT and RDX can be used as the explosive.

In the detonation, the carbon released due to partial incomplete combustion of the explosive is used as a raw material, and a nanodiamond crude product (containing nanodiamond agglutinate, soot, and oxides of metals, such as Fe, Co, and Ni, derived from the container and the like) is produced by the action of the pressure and energy of the shock wave generated by the explosion.

The acid treatment step involves allowing a strong acid to act on the nanodiamond crude product, for example, in an aqueous solvent to remove metal oxides. Examples of the strong acid include hydrochloric acid, hydrofluoric acid, sulfuric acid, nitric acid, and a mixture of these. The acid treatment temperature is, for example, from 70 to 150°C. The acid treatment time is, for example, from 0.1 to 24 hours. After the acid treatment, the solid content is preferably washed with water, for example, by decantation until the pH of the precipitation liquid reaches, for example, 2 to 3.

The oxidation treatment step involves removing graphite from the nanodiamond crude product using an oxidant. For the oxidant, for example, a mixed acid (particularly, the weight ratio of sulfuric acid/nitric acid is, for example, from 60/40 to 95/5) can be suitably used.

The amount of the oxidant (particularly, the mixed acid) to be used is, for example, from 20 to 40 parts by weight per part by weight of the nanodiamond crude product.

In the case of using the mixed acid as the oxidant, a catalyst may be used together with the mixed acid. The use of a catalyst can further improve the efficiency of graphite removal. Examples of the catalyst include copper(II) carbonate. The amount of the catalyst to be used is, for example, from about 0.01 to about 10 parts by weight per 100 parts by weight of the nanodiamond crude product.

The oxidation treatment temperature is, for example, from 100 to 200°C. The oxidation treatment time is, for example, from 1 to 24 hours.

The drying step involves evaporating a liquid content from the nanodiamond-containing solution prepared in the oxidation treatment step. For example, the step can be carried out using a spray dryer, an evaporator, a drying oven, or the like. A nanodiamond powder is produced through this step.

The surface functional group adjustment step involves adjusting a surface functional group of the nanodiamond powder produced through the drying step. This step includes a hydrogenation treatment and an oxygen oxidation treatment. These treatments can be selected and carried out depending on the intended purpose.

The hydrogenation treatment is a treatment of heating the nanodiamond powder in a hydrogen-containing gas atmosphere. The temperature condition of the hydrogenation treatment is preferably from 400 to 800°C. The hydrogen-containing gas may be a mixed gas containing an inert gas in addition to hydrogen. Examples of the inert gas include nitrogen, argon, carbon dioxide, and helium. The hydrogen concentration of the hydrogen-containing gas is preferably from 0.1 to 99.9 vol.%, more preferably from 0.5 to 50 vol.%, and more preferably from 1 to 10 vol.%. The hydrogenation treatment can remove a carboxy group or the like present on the nanodiamond surface.

The oxygen oxidation treatment is a treatment of heating the nanodiamond powder in an oxygen-containing gas atmosphere. The oxygen oxidation treatment temperature is, for example, from 250 to 500°C. The oxygen-containing gas is a mixed gas containing an inert gas in addition to oxygen. The oxygen concentration of the mixed gas is, for example, from 1 to 35 vol.%. The oxygen oxidation treatment can provide the nanodiamond surface with a functional group (e.g., a hydroxy group) having reactivity with glycidol.

When the nanodiamond produced through the surface functional group adjustment step is in the form of agglutinates (secondary particles), a disintegration treatment or a classification treatment is preferably carried out. Examples of the disintegration treatment include ultrasonication and bead milling treatment (e.g., bead milling treatment using zirconia beads).

The average particle size of the ND particle before surface modification is not particularly limited and is, for example, 100 nm or less, preferably 50 nm or less, more preferably 30 nm or less, and particularly preferably 10 nm or less. The lower limit of the average particle size of the ND particle is, for example, 0.1 nm. The average particle size of the surface-modified ND can be measured using a dynamic light scattering method.

Each aspect disclosed in the present specification can be combined with any other feature disclosed herein. The configurations, combinations thereof, or the like in each of the embodiments are exemplary, and additions, omissions, replacements, and other changes of the configurations may be made as appropriate without departing from the spirit of the present disclosure. In addition, each aspect of the invention according to the present disclosure is not limited by the embodiments or the following examples but is limited only by the claims.

### Examples

Hereinafter, the present disclosure will be described in more detail by way of examples, but the present disclosure is not limited by the examples.

### Example 1

### Preparation of detonation ND "DND"

A molded explosive including an electric detonator was placed inside a pressure-resistant vessel for detonation, and the vessel was sealed. The vessel was made of iron and had a capacity of 15 m³. 0.50 kg of a mixture of TNT and RDX was used as the explosive. The mass ratio of TNT to RDX (TNT/RDX) in the explosive was 50/50. The electric detonator was then triggered to detonate the explosive in the vessel (production of a DND, an ND by a detonation method). Then, the vessel was allowed to stand at room temperature for 24 hours to lower the temperatures of the vessel and its interior. After this cooling, a DND crude product (containing soot and DND particle agglutinates) was collected by scraping the DND crude product deposited on the inner wall of the vessel with a spatula.

The DND crude product produced by performing the process described above multiple times was subjected to an acid treatment. Specifically, 6 L of a 10 mass% hydrochloric acid was added to 200 g of the DND crude product to prepare a slurry, and the slurry was subjected to a heating treatment for 1 hour under reflux at normal pressure conditions. The heating temperature in this acid treatment was from 85 to 100°C. Next, after cooling, the solid content (containing a DND agglutinate and soot) was washed with water by decantation. The solid content was repeatedly washed with water by decantation until the pH of a precipitation liquid reached 2 from the low pH side.

An oxidation treatment was then performed. Specifically, to the precipitation liquid (containing the DND agglutinate) obtained through decantation after the acid treatment, 6 L of 98 mass% sulfuric acid and 1 L of 69 mass% nitric acid were added to form a slurry, and then the slurry was subjected to a heat treatment under reflux at normal pressure conditions for 48 hours. The heating temperature in this oxidation treatment was from 140 to 160°C. Next, after cooling, the solid content (containing the DND agglutinate) was washed with water by decantation. The supernatant liquid from the initial water washing was colored, and the solid content was repeatedly washed with water by decantation until the supernatant liquid became visually transparent.

Next, the precipitation liquid (liquid containing the DND agglutinate) obtained through the water-washing treatment described above was dried, to prepare a dry powder (DND agglutinate). The drying treatment in the drying step was performed to dry by evaporation using an evaporator.

Next, 4.5 g of the dry powder (DND agglutinate) prepared through the drying step described above was allowed to stand inside a furnace core tube of a gas atmosphere furnace (trade name "Gas Atmosphere Tube Furnace KTF045N1", available from Koyo Thermo Systems Co., Ltd.), and nitrogen gas was continuously passed through the furnace core tube at a flow rate of 1 L/min for 30 minutes. Then, the flowing gas was switched from nitrogen to a mixed gas of oxygen and nitrogen, and the mixed gas was continuously flowed through the furnace core tube at a flow rate of 1 L/min. The oxygen concentration of the mixed gas was 4 vol.%. After switching to the mixed gas, the temperature inside the furnace was raised to a temperature set for heating of 400°C. The temperature was raised at a rate of 10°C/min to 380°C, which was lower by 20°C than the temperature set for heating, and then at a rate of 1°C/min from 380°C to 400°C. Then, the oxygen oxidation treatment was carried out on the DND powder inside the furnace while the temperature condition inside the furnace was maintained at 400°C. The treatment time was 3 hours.

A hydrogenation step was then carried out using the gas atmosphere furnace described above. Specifically, the DND powder prepared through the oxygen oxidation step was placed inside the gas atmosphere furnace and nitrogen gas was continuously passed through the gas atmosphere furnace at a flow rate of 1 L/min for 30 minutes. Then, the flowing gas was switched from nitrogen to a mixed gas of hydrogen and nitrogen, and the mixed gas was continuously flowed through the furnace core tube at a flow rate of 1 L/min. The hydrogen concentration of the mixed gas was 2 vol.%. After switching to the mixed gas, the temperature inside the furnace was raised to a temperature set for heating of 600°C. The rate of temperature rise was 10°C/min. Then, the hydrogenation treatment was performed on the DND powder inside the furnace while the temperature inside the furnace was maintained at 600°C. The treatment time was 5 hours. The DND powder subjected to the hydrogenation treatment was obtained as described above.

A disintegration step was then performed. Specifically, 0.9 g of the DND powder obtained through the hydrogenation step described above and 29.1 mL of pure water were added to a 50-mL sample bottle and mixed together, to prepare about 30 mL of a slurry. After the pH was adjusted to 4 with 1 N hydrochloric acid, the slurry was subjected to ultrasonication. In the ultrasonication, the slurry was irradiated with ultrasonic waves for 2 hours using an ultrasonic irradiator (trade name "Ultrasonic Cleaner AS-3", available from AS ONE Corporation). Thereafter, bead milling was performed using a bead milling apparatus (trade name "Parallel 4-Tube Sand Grinder LSG-4U-2L", available from Aimex Co., Ltd.). Specifically, 30 mL of the slurry after the ultrasonic irradiation and zirconia beads having a diameter of 30 µm were charged in a 100-mL mill vessel (available from Aimex Co., Ltd.), and the vessel was sealed. Then, the apparatus was operated to perform bead milling. In this bead milling, the amount of charged zirconia beads is, for example, 33 vol.% of the capacity of the mill vessel, the rotation speed of the mill vessel was 2570 rpm, and the milling time was 2 hours.

Next, the slurry prepared through the disintegration step described above was subjected to a centrifugation treatment (classification operation) using a centrifuge. The centrifugal force in this centrifugation treatment was 20000 × g, and the centrifugation time was 10 minutes. Next, 10 mL of a supernatant of the DND-containing solution subjected to the centrifugation treatment was collected. Thus, a DND aqueous dispersion in which DND particles were dispersed in pure water was prepared. This aqueous dispersion had a solid content concentration of 2.1 mass% and a pH of 5.40. The median size (particle size D50) of the DND aqueous dispersion prepared as described above was 35.8 nm.

### Preparation of DND surface-modified with polyglycerol, "DND-PG"

An aqueous DND dispersion (D50 of DND: 4.5 nm, trade name "Dinnovare" ζ + Nanodiamond Aqueous Dispersion", available from Daicel Corporation) was concentrated under reduced pressure with an evaporator and further dried at 105°C for 2 hours. The resulting DND powder (1.0 g) was suspended in ethylene glycol (15.0 g), glycidol (45.1 g, 0.61 mol) was added dropwise to the suspension over 160 minutes, and the temperature of the mixture was maintained in a range from 95 to 100°C. The resulting black dispersion was stirred at the same temperature for 4 hours. Water (40 mL) was slowly added to the dispersion to decompose unreacted glycidol, and then the dispersion was diluted with water to 400 mL. The diluted dispersion was then concentrated to 20 mL or less with an ultrafiltration membrane (Ultracel Membrane, 30 kDa). The concentrate was diluted and concentrated again. This operation was repeated five times to wash the concentrate, to prepare a purified DND-PG in the form of a black aqueous dispersion. The yield was 100.0 g (content of the DND-PG was 4.10 wt.%, 4.10 g).

A portion of the dispersion was dried on a heated PTFE sheet, to prepare a sample for analysis. The analysis results are shown below.

FT-IR (diffuse reflection method, cm⁻¹): 3332, 2918, 2875, 1456, 1118, 1078.
¹H-NMR (500 MHz, D₂O): δ ppm 3.42, 3.50, 3.58, 3.75, 3.88.

TGA (in an air atmosphere, 20°C/min) measurement was carried out. As a result, the weight losses (%) were as follows.
· 374 to 530°C; 72.3%
· 530 to 798°C; 23.6%

From the results, the weight ratio of PG/DND (including free-PG impurities) was estimated to be 3.1.

### Removal of free-PG impurities by ultracentrifugation

The aqueous dispersion of the DND-PG (30.0 g, solid content concentration: 4.10%, content of DND-PG: 1.23 g) was ultracentrifuged (50000 rpm (183400 × g) for 2 hours) by ultracentrifugation (HIMAC CP 70MX Ultracentrifuge). About 20 mL of the supernatant was removed, and 20 mL of water was added to the remaining lower layer to produce a homogeneous dispersion, and the dispersion was further ultracentrifuged under the same conditions. This operation was repeated twice. The supernatant was removed, water was added to the remaining lower layer, and free-PG was removed, to prepare 30.8 g of a black aqueous dispersion of the DND-PG. The pH of 15.8 g of the dispersion was adjusted to 1.93, and then the dispersion was subjected to dilution and concentration with an ultrafiltration membrane (400 mL was concentrated to 10 mL or less). This operation was repeated five times to prepare 15.77 g of a black dispersion. The content of the DND-PG was 2.87 wt.% (0.45 g as solid content).

The results revealed that the content of the DND-PG in 30.8 g of the black aqueous dispersion of the DND-PG was 0.88 g, and the recovery rate of the DND-PG from the DND-PG aqueous dispersion before ultracentrifugation was 71.5 wt.%.

The zeta potential of the DND-PG aqueous dispersion at pH 8.1 was +18.0 mV.

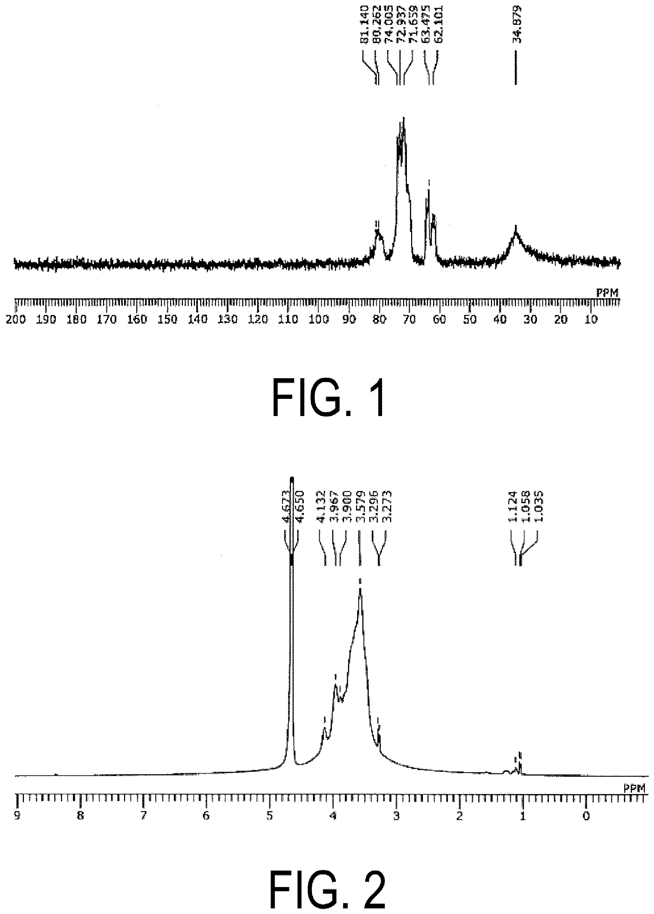

A portion of the resulting black aqueous dispersion of DND-PG was dried on a heated PTFE sheet, to prepare a sample. The resultant sample was used for ¹³C-NMR measurement of the DND-PG. The results are shown in FIG. 1.

TGA (20°C/min in an air atmosphere) measurement of the DND-PG was carried out. As a result, the weight losses (%) were as follows. From the results, the PG modification rate (PG/DND weight ratio) was estimated to be 2.0.
· 360 to 528°C: 63.6%
· 528 to 613°C: 31.8%

The structure of the resulting DND-PG was confirmed by FT-IR and solution ¹H-NMR. In the FT-IR spectrum, absorption due to O-H stretching at 3333 cm⁻¹, C-H asymmetric and symmetric stretching at 2918 and 2876 cm⁻¹, and C-O-C asymmetric and C-O stretching of the PG chain at 1118 and 1078 cm⁻¹ were observed. In the ¹H-NMR, signals at 3 to 4 ppm indicate that all hydrogen atoms on the PG chain are on carbons adjacent to oxygen-containing functional groups such as a hydroxy group and an ether bond.

### Preparation of DND-PG-COOH

12.47 g of water was added to 7.74 g of an aqueous dispersion of DND-PG (content of DND-PG: 0.25 g), and 0.4 M sodium acetate buffer (pH 4.7, 12.51 g) was further added thereto. Sodium chlorite (51.2 mg, purity: 81%, 0.46 mmol) and 4-acetamido-2,2,6,6-tetramethylpiperidine 1-oxyl free radical (4-acetamido-TEMPO, 13.5 mg, 0.063 mmol) were added thereto, and an aqueous sodium hypochlorite solution (37.5 uL, concentration: 12%, 0.060 mmol) was further added thereto, followed by stirring at 50°C for 24 hours. Ethanol was added to the reaction liquid to decompose excess oxidant, and then filtration with an ultrafiltration membrane (diluted with water to 400 mL and then concentrated to 10 mL or less) was performed twice. 6 M hydrochloric acid (2.0 mL) was added to the concentrate for acidification, and then filtration and washing with an ultrafiltration membrane were performed twice. The yield of the resulting DND-PG-COOH was 239 mg. Acid-base titration (indicator: phenolphthalein, titration with 0.05 M alcoholic KOH solution) of the DND-PG-COOH revealed that the -COOH content was 1.17 mmol/g.

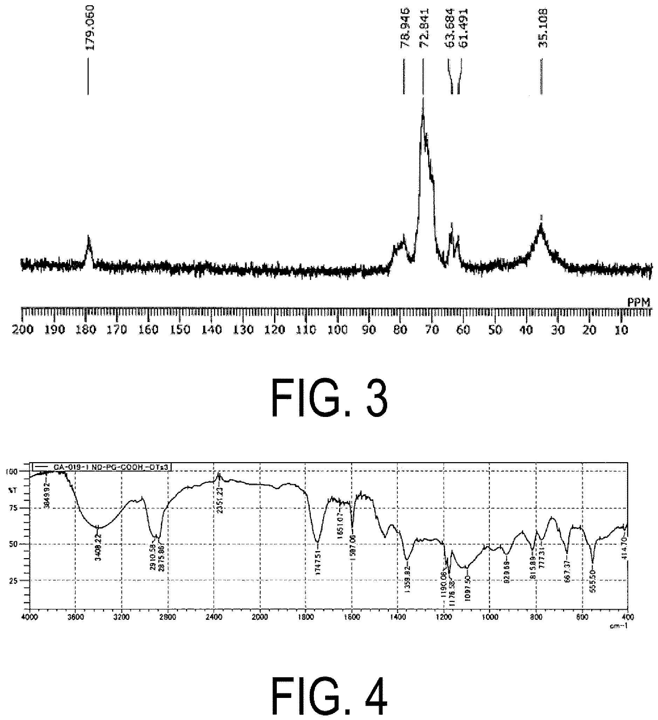

The analytical results of the resulting DND-PG-COOH are described below. FIGS. 2 and 3 illustrate the results.
FT-IR (KBr, diffuse reflection method, cm⁻¹): 3273, 2906, 2878, 1456, 1734, 1122, 1080.
¹H-NMR (500 MHz, 0.5% NaOD): δ ppm 4.13, 3.97, 3.90, 3.58.
¹³C-NMR (125 MHz, 0.5% NaOD): δ ppm 179.1, 78.9, 72.8, 63.7, 61.5, 35.1.
Elemental analysis: C; 59.36%, H; 5.63%, N; 0.73%, O; 34.16%

### Preparation of DND-PG(OTs)-COOH

10.6 g of an aqueous dispersion of DND-PG-COOH (content of DND-PG-COOH: 0.15 g) was dried and concentrated under reduced pressure. The residue was subjected to azeotropic dehydration twice with pyridine (10 mL), and then the residue was dispersed in pyridine (5.0 mL). p-Toluenesulfonyl chloride (TsCl, 283.4 mg, 1.49 mmol) and N,N-dimethylaminopyridine (8.1 mg, 0.066 mmol) were added to the dispersion under ice cooling, and the mixture was stirred at room temperature for 14 hours. Water was added to the reaction liquid, and the mixture was centrifuged (30000 g × 10 min). The precipitate was dispersed in a small amount of tetrahydrofuran (THF), and water was added to the dispersion for reprecipitation, followed by centrifugation. This operation was repeated twice for washing of the precipitate. The precipitate was further dispersed in a small amount of THF, and toluene was added to the dispersion for reprecipitation, followed by centrifugation. This operation was performed twice. The precipitate was dried by heating at 40°C under reduced pressure. Thus, 194.3 mg of DND-PG(OTs)-COOH was prepared in the form of a black solid.

The analytical results of the resulting DND-PG(OTs)-COOH are shown below and in FIG. 4.

FT-IR (KBr, diffuse reflection method, cm⁻¹): 3408, 2911, 2875, 1747, 1597, 1359, 1176, 1097, 929, 815, 687, 556.

### Preparation of DND-PG(BSH)-COOH

Disodium mercaptoundecahydrododecaborate (disodium mercaptoundecahydrododecaborate-10B, BSH, 91.1 mg, 0.43 mmol) was dissolved in a mixed solution of water (2.0 mL) and a 10% (w/v) aqueous sodium hydroxide solution (0.18 mL), acrylonitrile (0.05 mL, 0.76 mmol) was added to the solution, and the mixture was stirred at room temperature for 3 hours. The pH was adjusted to from 7 to 8 by adding 1 M hydrochloric acid (0.44 mL), and then the mixture was dried and concentrated under reduced pressure. Acetonitrile (3.0 mL) was added to the residue, and the mixture was dried and concentrated. This operation was performed twice to remove water. Subsequently, the resultant product was dissolved in N,N-dimethylformamide (DMF, 2.0 mL) to prepare a solution of an acrylonitrile adduct of BSH.

DND-PG(OTs)-COOH (estimated to have 129.5 mg (0.19 mmol) of -OTs groups) was dispersed in DMF (4.0 mL), and the whole amount of the solution of the acrylonitrile adduct of BSH prepared through the above operation was added to the dispersion. The reaction liquid was stirred with heating at 90°C for 48 hours and then at 120°C for 2 hours. In the course of the reaction, a solution of an acrylonitrile adduct of BSH prepared in the same manner as described above from BSH (39.5 mg, 0.19 mmol) was added, and tetrabutylammonium iodide (21.4 mg, 0.058 mmol) was also added. After cooling, water (5.0 mL) was added to the reaction liquid, and the mixture was centrifuged (30000 g × 10 min). The precipitate was suspended in water and washed by centrifugation. Then, DMF (3.0 mL) and 2% aqueous sodium hydroxide solution (1.25 mL) were added to the precipitate, and the mixture was stirred at 60°C for 4.5 hours. Water (15 mL) and 6 M hydrochloric acid (0.20 mL) were added to the reaction liquid, and the mixture was ultracentrifuged (183400 g × 10 min). Water (15 mL) was added to the precipitate for dispersion, and the precipitate was washed by ultracentrifugation to prepare DND-PG(BSH)-COOH.

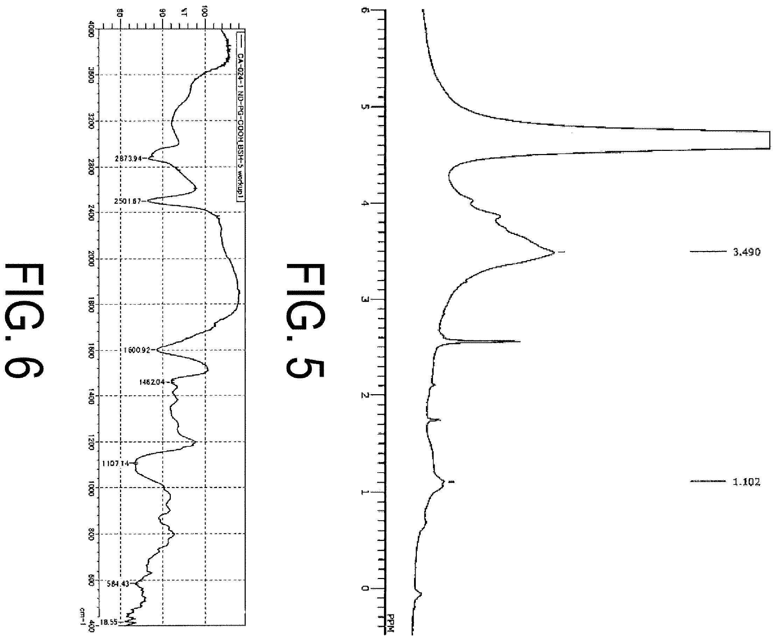

The results of ¹H-NMR and FT-IR of the resulting DND-PG(BSH)-COOH are shown below and in FIGS. 5 and 6.

In ¹H-NMR of the DND-PG(BSH)-COOH, a multiplet attributed to BH of BSH (boron cluster) was observed around 1.0 ppm. In addition, a peak attributed to BH stretching vibration was observed at 2501 cm⁻¹ in FT-IR.

### Preparation of DND-PG(BSH)-PBA

DND-PG(BSH)-COOH (net content: about 39 mg) derived from 64.9 mg of DND-PG(OTs)-COOH was dispersed in 3.0 mL of water, and 3.0 mL of methanol was added to the dispersion. 3-Aminophenylboronic acid hydrate (7.3 mg, 0.047 mmol) and 4-methylmorpholine (25 uL, 0.23 mmol) were added to the dispersion, 4-(4,6-dimethoxy-1,3,5-triazin-2-yl)-4-methylmorpholinium chloride (DMT-MM, 12.8 mg, 0.046 mmol) was further added to the mixture, and the mixture was stirred at room temperature for 13 hours. 6 M hydrochloric acid was added to the reaction liquid for acidification, followed by centrifugation (30000 g × 10 minutes). The precipitate was dispersed in 10 mL of water and 0.20 mL of 1 M aqueous sodium hydroxide solution, and the dispersion was concentrated by centrifugal filtration using Amicon Ultra15. The residue on the filter was dispersed in 15 mL of water and washed by centrifugal filtration. Then, the resultant product was washed once with a mixture of 10 mL of water and 1 mL of dimethyl sulfoxide, and then washed three times with water. The volume of the target product was adjusted to 5.0 mL with water, followed by cotton plug filtration and removal of insoluble matter, to produce DND-PG(BSH)-PBA.

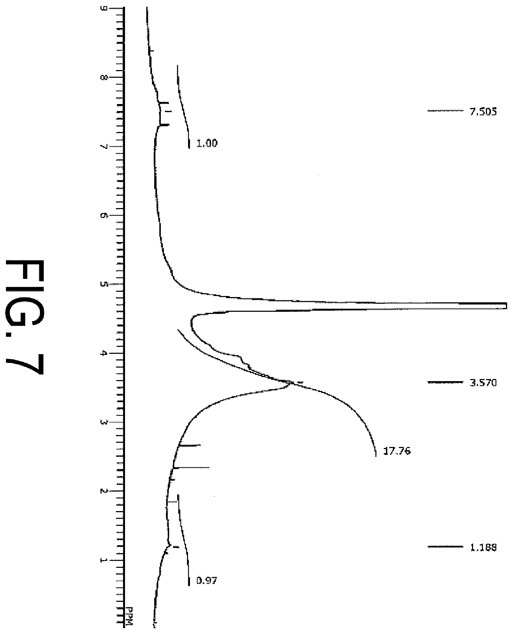

The results of ¹H-NMR of the resulting DND-PG(BSH)-PBA are shown below and in FIG. 7.

¹H-NMR: Signals attributed to BH of the boron cluster were observed around 1.0 ppm, and signals attributed to phenyl groups of phenylboronic acid were observed around 7.5 ppm.

¹H-NMR analysis of each sample was performed using an ECX500 NMR spectrometer (JEOL). In addition, the FT-IR analysis was performed using a Fourier transform infrared spectrophotometer "IRTracer" (available from Shimadzu Corporation) equipped with a heat-vacuum stirring reflection "Heat Chamber Type-1000°C" (available from ST Japan Inc.). The same shall apply to the experiments described below.

### Example 2

DND-PG-COOH was prepared in the same manner as in Example 1.

### Preparation of DND-PG(OTs)-COOH

13.5 g of an aqueous dispersion of DND-PG-COOH (content of DND-PG-COOH: 0.40 g, 2.95% (w/w), PG/DND weight ratio = 3.85, COOH content: 1.01 mmol/g), 2.45 mL of water, and 8M NaOH (3.0 mL, 24 mmol) were added, and the mixture was cooled in an ice bath. A solution of 0.58 g (3.0 mmol) of p-toluenesulfonyl chloride (TsCl) in 3.6 mL of tetrahydrofuran (THF) was added intermittently to the mixture for about 1 hour with vigorous stirring. The reaction mixture was stirred for additional 2 hours under ice cooling conditions, and then the mixture was acidified by addition of 4.0 mL of 6 M hydrochloric acid and stirred for several hours. The resulting reaction mixture was centrifuged (3000 rpm, 30 min), and a small amount of THF (from 2 to 3 mL) and water (from 10 to 15 mL) were added to the precipitate for washing the precipitate three times. Ultracentrifugation (183400 g × 10 min) was performed depending on the dispersion state of the supernatant to give a precipitate. The precipitate was dispersed in 20 mL of N,N-dimethylformamide (DMF) and ultracentrifuged (183400 g × 30 min). This operation was repeated twice as solvent replacement to prepare 16.5 g of a DMF dispersion of DND-PG(OTs)-COOH. The yield of DND-PG(OTs)-COOH was 0.27 g, and the concentration was 1.4% (w/w).

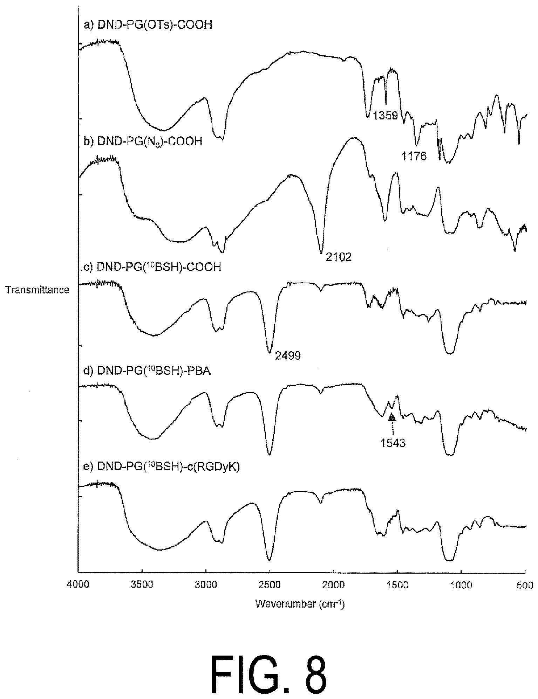

The analysis results of the resulting DND-PG(OTs)-COOH are shown below. FIG. 8(a) illustrates the results of the FT-IR spectrum.

Elemental analysis: C; 56.85%, H; 6.53%, N; 0.51%, O; 29.08%, S; 4.08%

### Preparation of DND-PG(N₃)-COOH

196 mg (3.0 mmol) of sodium azide (NaN₃), 3.0 mL of water, and 0.30 mL of 1 M NaOH (0.30 mmol) were added to 13.8 g of a DMF dispersion of DND-PG(OTs)-COOH (content of DND-PG(OTs)-COOH: 0.20 g, 1.48% (w/w)). The resulting reaction mixture was stirred at 60°C for 0.5 hours and then the temperature was raised to 90°C and stirred for 18 hours. The resulting suspension was ultracentrifuged (183400 g × 2 hours) and the precipitate was washed three times with water by dilution and ultracentrifugation (183400 g × 30 min). The precipitate was dispersed in 30.0 g of water. The yield of DND-PG(N₃)-COOH was 0.17 g, and the concentration was 0.57% (w/w).

The analytical results of the resulting DND-PG(N₃)-COOH are shown below. FIG. 8(b) illustrates the results of the FT-IR spectrum.

Elemental analysis: C; 55.28%, H; 5.54%, N; 5.46%, O; 30.96%

### Preparation of ¹⁰BSH(Pgy)

To 4.0 mL of water was added 192 µL of 10 wt.% aqueous NaOH solution (content of NaOH: 19.2 mg, 0.48 mmol), and the mixture was vacuum degassed under ultrasonic irradiation. 100.9 mg (0.48 mmol) of sodium mercaptoundecahydro-closo-dodecaborate (¹⁰B) (¹⁰BSH) was dissolved in the aqueous NaOH solution prepared in the above-described operation, and then 60 µL of acrylonitrile (0.92 mmol) was added to the solution. The mixture was stirred at room temperature for 5 hours in a nitrogen atmosphere. The stirred mixture was washed three times with about 10 mL of ethyl acetate (AcOEt), and the water layer was evaporated to give 204 mg of S-(2-cyanoethyl)thioundecahydro-closo-dodecaborate (¹⁰B) (¹⁰BSH(AN)) in the form of a colorless oily product.

The resulting ¹⁰BSH(AN) was dissolved in a mixture of 20.0 mL of acetonitrile (MeCN) and 5.0 mL of water, and 199 µL of a mixed solution of propargyl bromide (314 mg, 2.64 mmol) in MeCN (2.0 mL)/water (0.50 mL) was slowly added, followed by stirring at room temperature overnight. The reaction product was evaporated and dried, MeCN was added to the residue, and evaporated again to remove residual water. About 10 mL of MeCN was added to the residue, and the insoluble salt was removed by filtration. The filtrate was concentrated to give 170 mg of a crude product of S,S-[(2-cyanoethyl)-(2-propynyl)]sulfonioundecahydro-closo-dodecaborate (¹⁰B) (¹⁰BSH(AN)(Pgy)) in the form of a pale yellow oily product.

The resulting crude ¹⁰BSH(AN)(Pgy) was dissolved in 2.0 mL of acetone, and 1.04 mL (0.96 mmol) of 10 wt.% methanol solution of tetramethylammonium hydroxide was added thereto, to give a white precipitate, followed by collection of the precipitate by filtration. The residue was washed with a small amount of acetone and dried under vacuum at room temperature to give 151 mg (theoretical yield: 167 mg) of bis-tetramethylammonium S-(2-propynyl)thioundecahydro-closo-dodecaborate (¹⁰B) (¹⁰BSH(Pgy) 2TMA) in the form of an off-white to pale color powder. The product may contain an inorganic salt (NaBr) as an impurity but was used in the subsequent click reaction without further purification. The above product is referred to as "crude ¹⁰BSH(Pgy)·2TMA".

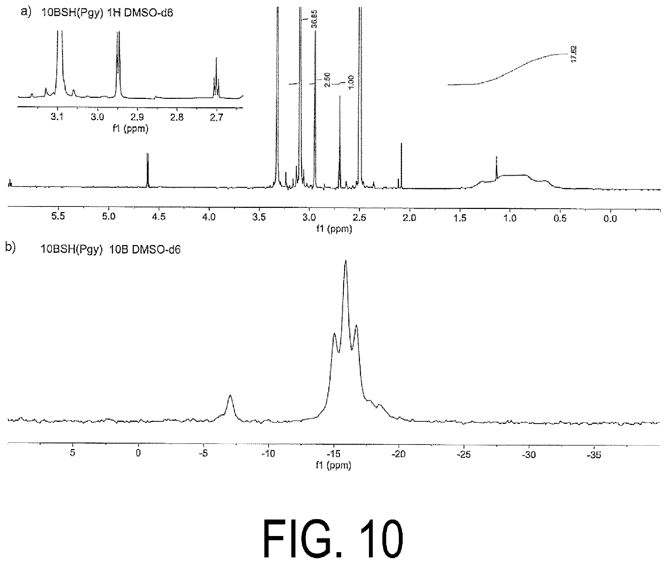

FIG. 10(a) illustrates the ¹H-NMR spectrum of ¹⁰BSH(Pgy) and FIG. 10(b) illustrates the ¹⁰B-NMR spectrum of ¹⁰BSH(Pgy). The measurement solvent is DMSO-d6. The synthesis scheme of ¹⁰BSH(Pgy) is shown below.

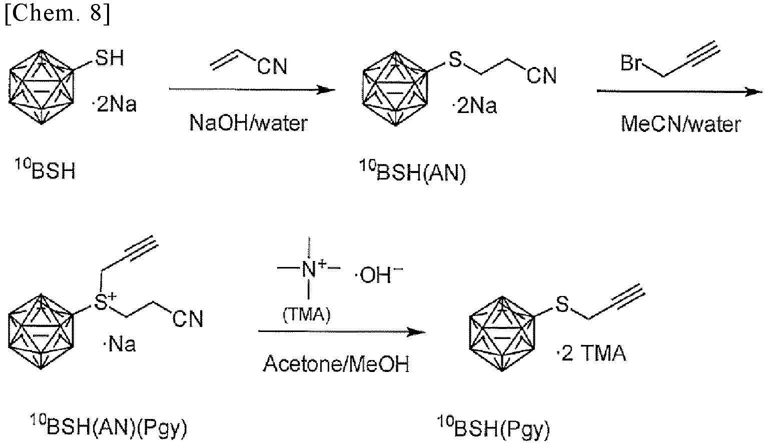

### Preparation of DND-PG(¹⁰BSH)-COOH

4.0 mL of acetonitrile (MeCN) and 2.5 mL of 0.4 M phosphate buffer (pH 7.4) were added to 8.84 g of an aqueous dispersion of DND-PG(N₃)-COOH (0.57% (w/w), content of DND-PG(N₃)-COOH: 50.1 mg), and a mixture of 42.2 mg (0.21 mmol) of sodium ascorbate and 50.3 mg of crude ¹⁰BSH(Pgy)·2TMA was vacuum degassed by ultrasonication. An aqueous CuSO₄ solution (content of CuSO₄: 14.2 mg, 0.088 mmol, water: 0.50 mL) was slowly added to the mixture, followed by stirring at room temperature under a nitrogen atmosphere. The reaction was monitored by FT-IR of the collected reaction liquid sample (increase and decrease of azido N₃ and B-H absorption peaks at 2102 cm⁻¹ and 2503 cm⁻¹, respectively). In the course of the reaction, 24.8 mg (total amount of three additions) of crude ¹⁰BSH(Pgy)·2TMA, 10.3 mg of sodium ascorbate, and 3.4 mg of CuSO₄ were added from 22 to 30 hours after the initiation of the reaction, and the mixture was further stirred for 2 days. The resulting dark-brown dispersion was ultracentrifuged (183400 g × 30 minutes) and the precipitate was washed twice with 10 mL of water and 10 mL of an aqueous solution (0.3%) of ethylenediamine-N,N,N',N'-tetraacetic acid disodium salt (EDTA·2Na), and twice with 10 mL of water by using a centrifugal filter (Amicon Ultra, 30 kDa). The concentrate on the filter was diluted with water to give 10.1 g of a brown dispersion product. The concentration was 0.50% (w/w) based on the weight after lyophilization, and the yield of DND-PG(¹⁰BSH)-COOH was 50.5 mg.

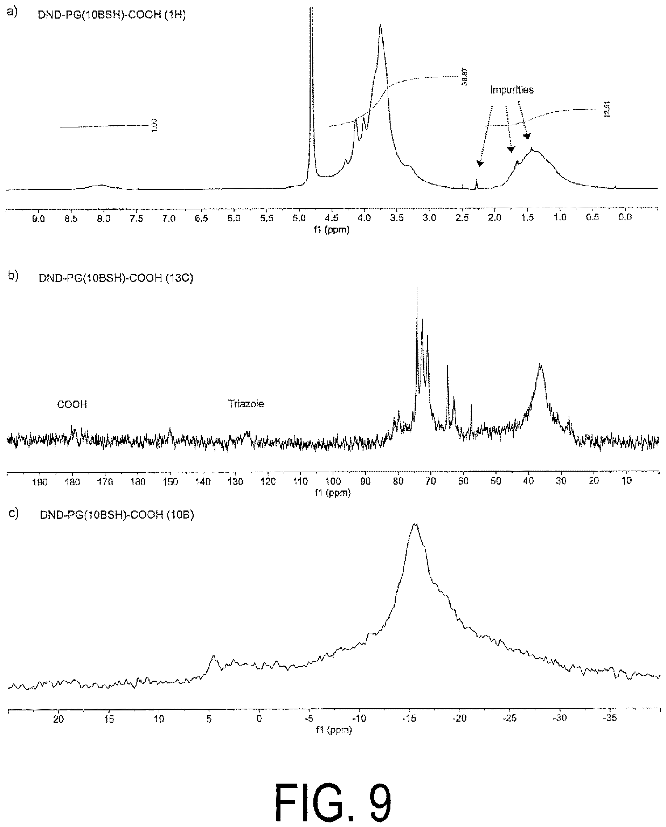

The analytical results of the resulting DND-PG(¹⁰BSH)-COOH are shown below. FIG. 8(c) illustrates the results of the FT-IR spectrum. FIG. 9(a) illustrates the ¹H-NMR spectrum, FIG. 9(b) illustrates the ¹³ C-NMR spectrum, and FIG. 9(c) illustrates the ¹⁰B-NMR spectrum. The measurement solvent is D₂O.

Elemental analysis: C; 48.22%, H; 5.33%, N; 3.78%

The boron content was 7.97% as measured by ICP emission spectrometry (ICP-AES) using SPS3100 (available from SII Nano Technology Inc.) after treating the sample with alkali fusion.

### Preparation of DND-PG(¹⁰BSH)-PBA

To 5.0 g of an aqueous dispersion of DND-PG(¹⁰BSH)-COOH (content of DND-PG(¹⁰BSH)-COOH: 25.0 mg, 0.50% (w/w)) were added 2 mL of 0.1 M aqueous solution of 2-(N-morpholino)ethanesulfonic acid (MES) (pH 4.5), 0.22 mL of a DMF solution of 3-aminophenylboronic acid monohydrate (content of 3-aminophenylboronic acid monohydrate: 2.2 mg, 1.0 wt.%, 0.014 mmol), and 0.50 mL of an aqueous solution of 1-(3-dimethylaminopropyl)-3-ethylcarbodiimide hydrochloride (EDC) (content of 1-(3-dimethylaminopropyl)-3-ethylcarbodiimide hydrochloride: 5.0 mg, 0.026 mmol), and the mixture was stirred at room temperature for 15 hours. The pH of the reaction mixture was adjusted to 10 with 1 M NaOH, and the mixture was stirred for 45 minutes. The dispersion was filtered through a centrifugal filter (Amicon Ultra 30 kDa) and then washed five times with water. The concentrate on the filter was diluted with water to give 4.91 g of a brown dispersion. The concentration of DND-PG(¹⁰BSH)-PBA was 0.50% (wt/wt) based on the weight after lyophilization, and the yield was 24.6 mg.

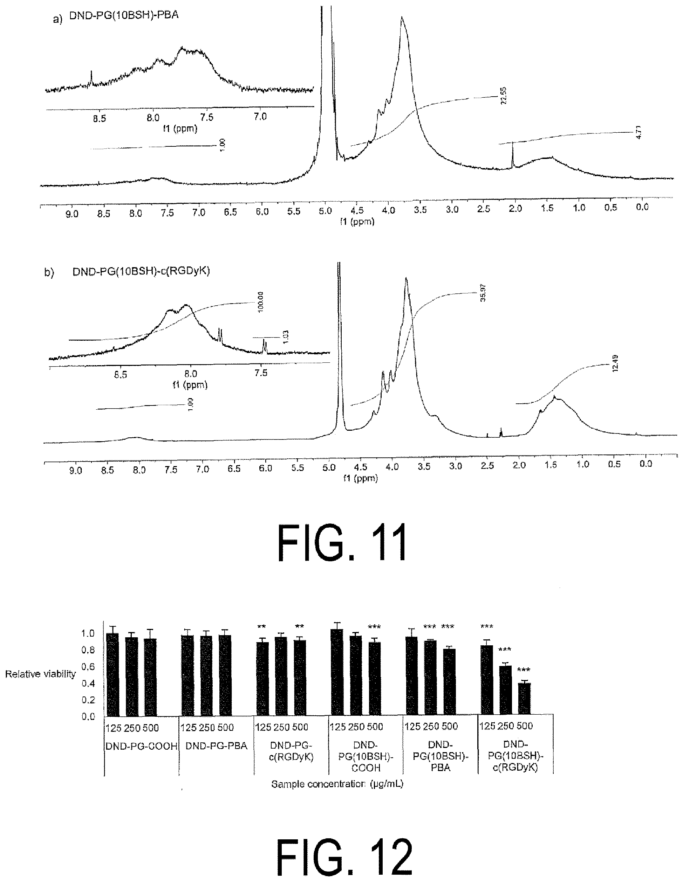

The analytical results of the resulting DND-PG(¹⁰BSH)-PBA are shown below. FIG. 8(d) illustrates the results of the FT-IR spectrum. FIG. 11(a) illustrates the results of the ¹H-NMR spectrum. The measurement solvent is D₂O.

Elemental analysis: C; 51.35%, H: 5.10%, N; 4.16%

From a change in nitrogen content in the elemental analysis, the amount of PBA groups introduced was estimated to be 0.40 mmol/g.

### Preparation of DND-PG-PBA

DND-PG-PBA was prepared in the same manner as described above in "Preparation of DND-PG(¹⁰BSH)-PBA" except that DND-PG-COOH was used in place of DND-PG(¹⁰BSH)-COOH.

### Preparation of DND-PG(¹⁰BSH)-c(RGDyK)

To 3.4 g of an aqueous dispersion of DND-PG(¹⁰BSH)-COOH (content of DND-PG(¹⁰BSH)-COOH: 16.9 mg, 0.50% (w/w)) were added 2 mL of 0.4 M phosphate buffer (pH 7.4), an aqueous N-hydroxysuccinimide solution (1.0 wt.%, 0.47 mL, content of N-hydroxysuccinimide: 4.7 mg), and an aqueous solution of 1-(3-dimethylaminopropyl)-3-ethylcarbodiimide hydrochloride (EDC) (1.0%, 0.39 mL, content of 1-(3-dimethylaminopropyl)-3-ethylcarbodiimide hydrochloride: 3.9 mg), and the mixture was stirred at room temperature for 30 minutes. Then, 7.4 mg of cyclic pentapeptide cyclo-(L-Arg-Gly-L-Asp-D-Tyr-L-Lys) (c(RGDyK)) was added, followed by stirring at room temperature for 24 hours. For the reaction mixture, the dispersion product was filtered through a centrifugal filter (Amicon Ultra 30 kDa) and then washed six times with water. The concentrate on the filter was diluted with water to give 1.70 g of a brown dispersion. The concentration of DND-PG(¹⁰BSH)-c(RGDyK) was 0.85% (wt/wt) based on the weight after lyophilization, and the yield was 14.5 mg. The introduction of c(RGDyK) was confirmed by the presence of two doublets (signals of Tyr aromatic ring) detected at 7.48 ppm and 7.78 ppm in ¹H-NMR (FIG. 11(b)). From the integral value of the Tyr signals, the amount of c(RGDyK) introduced was estimated to be 0.0053 mmol/g.

The analysis results of the resulting DND-PG(¹⁰BSH)-c(RGDyK) are shown below. FIG. 8(e) illustrates the results of the FT-IR spectrum. FIG. 11(b) illustrates the results of the ¹H-NMR spectrum. The measurement solvent is D₂O.

### Preparation of DND-PG-c(RGDyK)

DND-PG-c(RGDyK) was prepared in the same manner as described above in "Preparation of DND-PG(¹⁰BSH)-c(RGDyK)" except that DND-PG-COOH was used in place of DND-PG(¹⁰BSH)-COOH.

The synthesis scheme of Example 2 is shown below.

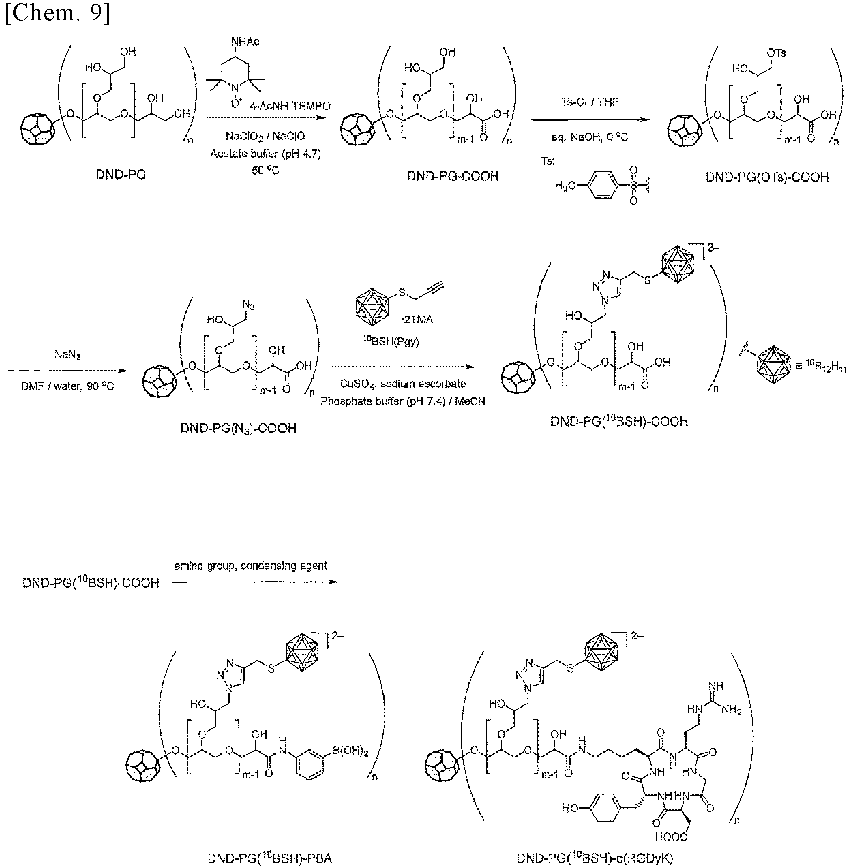

DND-PG-COOH produced in Example 1 and DND-PG-PBA, DND-PG-c(RGDyK), DND-PG(¹⁰BSH)-COOH, DND-PG(¹⁰BSH)-PBA, and DND-PG(¹⁰BSH)-c(RGDyK) produced in Example 2 (hereinafter may be referred to as "nanoparticles") were evaluated as described below.

### Evaluation 1: Cytotoxicity test

B16 mouse melanoma cells were seeded in a 96-well microplate at 3 × 10³ cells/well and cultured in 100 µL of Dulbecco's Modified Eagle Medium (DMEM, glucose: 4.5 g/L, 10% fetal bovine serum (FBS), and 1% 100 × penicillin-streptomycin-amphotericin B) in a CO₂ incubator at 37°C for 3 days. The medium (100 µL) was replaced, phosphate buffered saline (PBS, pH 7.4, control) or a PBS dispersion of nanoparticles (25 µL for 2500, 1250, and 625 µg/mL, concentration in the culture liquid: 500, 250, and 125 µg/mL, respectively) was added, and the cells were cultured for additional 24 hours. After the cells were washed with the medium (twice) and PBS, 100 µL of the medium was added, and 10 µL of CCK-8 (a mixture of 2-(2-methoxy-4-nitrophenyl)-3-(4-nitrophenyl)-5-(2,4-disulfophenyl)-2H-tetrazolium monosodium salt (WST-8) and 1-methoxy-5-methylphenazinium methyl sulfate (1-methoxy PMS), available from Dojindo Laboratories) was added. After the elapse of 0.5 hours, the absorbance of each well at 450 nm was measured using a microplate reader (FIG. 12).

### Evaluation 2: Confirmation of BNCT effect by thermal neutron irradiation on cell

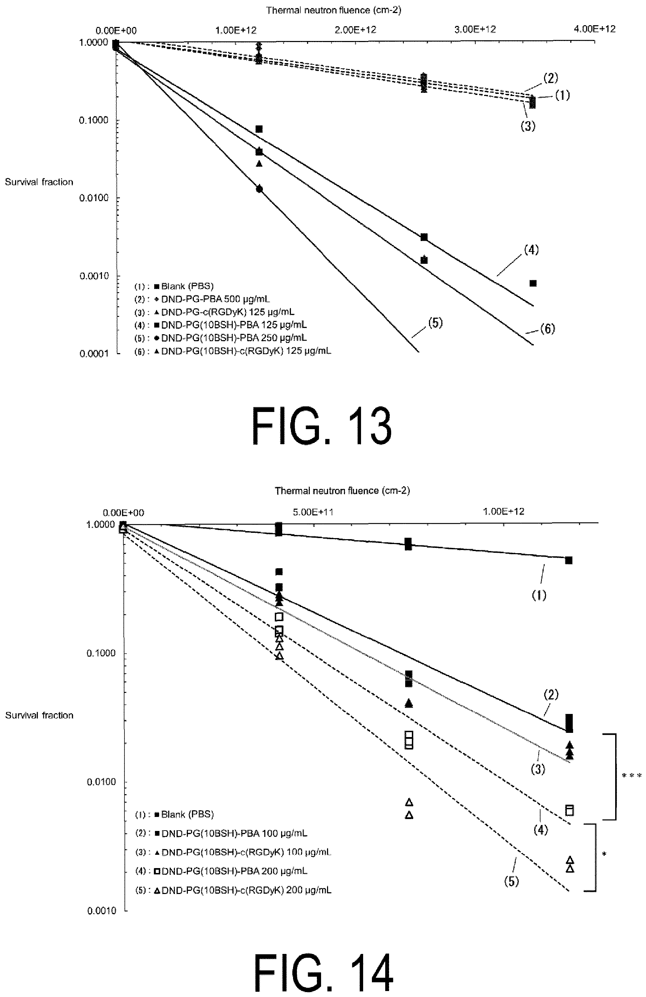

The BNCT effect of the nanoparticles was evaluated by thermal neutron irradiation of B16 mouse melanoma cells. The concentration of the nanoparticles in the sample was determined from the results of the cytotoxicity test described above. Regarding the concentrations of the nanoparticles, the concentration of DND-PG-PBA is 500 µg/mL (FIG. 13). The concentration of DND-PG-c(RGDyK) is 125 µg/mL (FIG. 13). The concentrations of DND-PG(¹⁰BSH)-PBA are 125 and 250 µg/mL (FIG. 13) and 100 and 200 µg/mL (FIG. 14). The concentrations of DND-PG(¹⁰BSH)-c(RGDyK) are 125 µg/mL (FIG. 13) and 100 and 200 µg/mL (FIG. 14).

B16 mouse melanoma cells (8.8 × 10⁵ or 1.0 × 10⁶ cells) were seeded in a 100-mm ϕ dish containing 10 mL of a DMEM medium (supplemented with 4.5 g/L of glucose, 10% FBS, and antibiotic/antimycotic) and cultured in a CO₂ incubator at 37°C for 2 days. The medium (10 mL, supplemented with or without FBS) was replaced once, and PBS or a PBS dispersion of nanoparticles (1.0 mL) was added to give the above-described predetermined concentrations. After culturing in a CO₂ incubator at 37°C for from 22 to 24 hours, the cells were washed with PBS and treated with trypsin (0.5%, 2.0 mL) at 37°C for 5 minutes, and the cells were detached. After termination of the trypsin treatment with a medium (containing FBS), the number of cells was counted, the concentration was adjusted to 100000 cells/ mL, and the cell suspension was dispensed into four plastic tubes (1 mL each). The thermal neutron beam was irradiated at three irradiation doses (for example, irradiation times of 5 minutes, 10 minutes, 15 minutes, and no irradiation (i.e., control)). In the research reactor KUR of Institute for Integrated Radiation and Nuclear Science, Kyoto University, the thermal neutron fluence (reaching 4.09 × 10¹¹, 7.48 × 10¹¹, and 1.17 × 10¹² neutrons/cm² at the irradiation times of 5 minutes, 10 minutes, and 15 minutes, respectively, during the operation with 1 MW of power) was determined by averaging the gold foil attached to the surface of the tube in the incident direction of thermal neutrons. A predetermined number of cells were seeded (from 300 to 10000 cells/dish) in a 60-mm ϕ dish containing 5 mL of a medium (supplemented with FBS). After culturing in a CO₂ incubator at 37°C for from 9 to 11 days, the resulting colonies were fixed with 70% ethanol and stained with a 0.1% crystal violet solution. The number of colonies was counted by visual inspection (naked eyes).

As a result, colony growth equivalent to that in the control group was observed in the nanoparticles not containing ¹⁰BSH, and it was confirmed that the nanoparticles were not effective in BNCT. In contrast, the nanoparticles containing ¹⁰BSH formed only few colonies. Furthermore, no or few colonies were formed at high concentrations or high irradiation doses (FIG. 13).

Also, at low concentrations or low irradiation doses, all the samples containing the ¹⁰BSH moiety showed good BNCT effects with thermal neutrons at 1.17 × 10¹² cm⁻², i.e., a viability of less than 0.01 at a nanoparticle concentration of 200 µg/mL (FIG. 14). The dependence between the concentration and the fluence was also observed. The slope difference between DND-PG(¹⁰BSH)-PBA and DND-PG(¹⁰BSH)-c(RGDyK) at 200 µg/mL was significant at the same concentration.

### Evaluation 3: Observation of cell introduction experiment of nanoparticle by transmission electron microscopy (TEM)

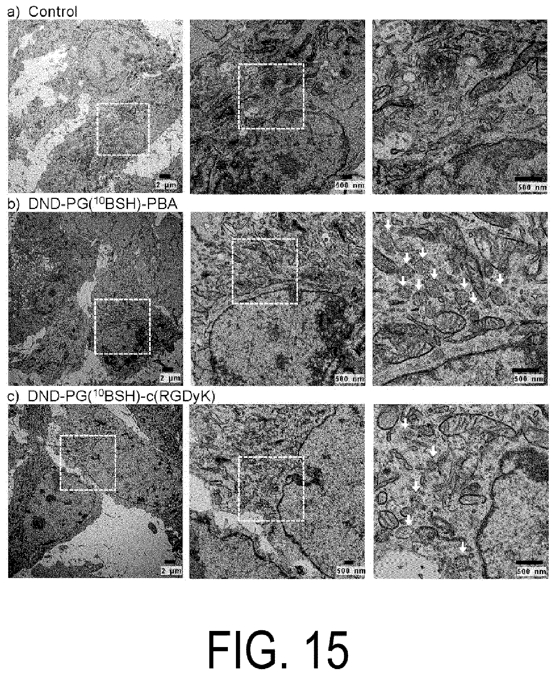

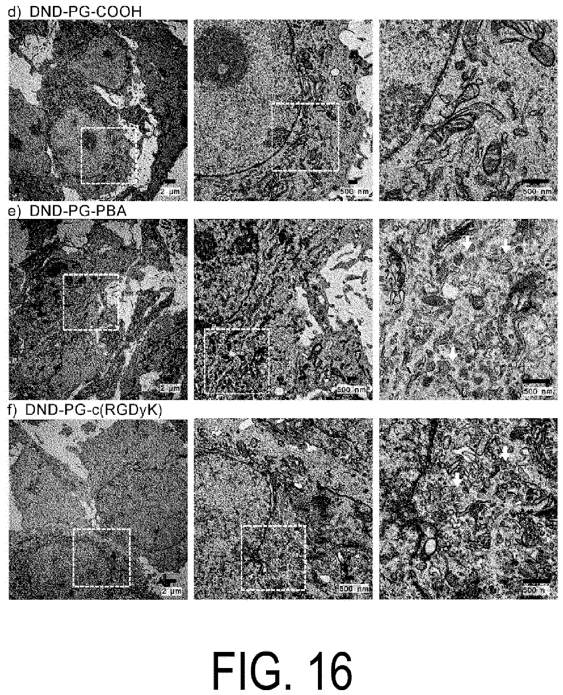

B16 cells were seeded on a chamber slide (Nagel Nunc177445) at 2.5 × 10⁴ cells/well (3.0 × 10⁴ cells/cm²) in 400 µL of a DMEM (containing 4.5 g/L of glucose and 10% FBS) medium. After culturing in a CO₂ incubator at 37°C for 3 days, the culture liquid (400 µL) was replaced once, PBS (control) or a PBS dispersion of nanoparticles having a predetermined concentration was added, and the cells were cultured for additional 24 hours. The cells were washed with medium (twice) and PBS, and the cells were fixed with a mixture of 25% glutaraldehyde and 4% paraformaldehyde 0.1 M phosphate buffer (1/10 (v/v)) in a refrigerator for several days. The fixed cells were then retained at 4°C with 1.5% potassium ferrocyanide followed by 2% osmium tetroxide in deionized water (DW). After the elapse of 1 hour, the cells were washed with DW and fixed with 2% osmium tetroxide in DW for 1 hour at room temperature. The sample was then stained overnight in a solution of 4% uranyl acetate in DW and then washed with DW. Thereafter, the sample was further stained with the Walton's lead aspartate solution for 2 hours, dehydrated in a dilution series of ethanol (60%, 70%, 80%, 90%, 99%, and 100%), and embedded in Epon 812. A thin section was prepared using an ultramicrotome (Leica UC7). The section was stained with uranyl acetate and lead citrate, and introduction of the nanoparticles into the cells was confirmed by TEM observation using H-7650 (transmission electron microscope, available from Hitachi High-Technologies Corporation) (FIGS. 15 and 16). In the figures, white arrows indicate the presence of vesicles due to the nanoparticles introduced into the cells.

FIG. 15 illustrates TEM images when a) the control, b) DND-PG(¹⁰BSH)-PBA, and c) DND-PG(¹⁰BSH)-c(RGDyK) were introduced into B16 cells. FIG. 16 illustrates TEM images when d) DND-PG-COOH, e) DND-PG-PBA, and f) DND-PG-c(RGDyK) were introduced into B16 cells.

In the TEM images of b) and c) regarding the "nanoparticles containing a boron cluster-containing group and a group capable of recognizing a cancer cell", vesicles indicating introduction of the corresponding nanoparticles into the cells were significantly observed. In the TEM images of e) and f) regarding the "nanoparticles containing a group capable of recognizing a cancer cell", vesicles indicating introduction of the corresponding nanoparticles into the cells were significantly observed. In contrast, the TEM image of d) regarding the "nanoparticles containing no group capable of recognizing a cancer cell", no introduction of the corresponding nanoparticles into the cells was determined. Comparison of b) and c) with e) and f) indicates that the amount of the corresponding nanoparticles introduced into the cells is larger. This is considered to be due to contribution of non-specific introduction of the boron cluster (¹⁰BSH) into the cancer cells.

From Evaluations 1 to 3 (particularly Evaluations 2 and 3) described above, it was confirmed that the nanodiamond particles having a boron cluster (¹⁰BSH)-containing group and a group capable of recognizing a cancer cell (PBA or c(RGDyK)) recognized a specific cancer cell and were introduced into the cell, and the nanodiamond particles exhibited the BNCT effect due to the introduction into the cancer cell (FIGS. 13 and 14, and FIG. 15 b) and c)). It was also confirmed that the nanodiamond particles not containing a boron cluster (¹⁰BSH)-containing group but containing a group capable of recognizing a cancer cell (PBA or c(RGDyK)) recognized a specific cancer cell and were introduced into the cell, but the nanodiamond particles did not exhibit the BNCT effect (FIG. 13, and FIG. 16 e) and f)).

To summarize the above, configurations and variations of the present disclosure are additionally described below.
[1] A surface-modified nanodiamond containing:
   a nanodiamond particle; and
   a boron cluster-containing group and a group capable of recognizing a cancer cell as surface modification groups of the nanodiamond particle.
[2] The surface-modified nanodiamond according to [1], wherein the boron cluster-containing group and the group capable of recognizing a cancer cell are present in the same surface modification group or different surface modification groups.
[3] The surface-modified nanodiamond according to [1] or [2], further containing a hydrophilic polymer chain-containing group as a surface modification group of the nanodiamond particle.
[4] The surface-modified nanodiamond according to [3], wherein a hydrophilic polymer chain in the hydrophilic polymer chain-containing group is a polyglycerol chain.
[5] The surface-modified nanodiamond according to [3] or [4], wherein the boron cluster-containing group, the group capable of recognizing a cancer cell, and the hydrophilic polymer chain-containing group are present in the same surface modification group or different surface modification groups.
[6] The surface-modified nanodiamond according to any one of [1] to [5], wherein the boron cluster-containing group is bonded to the nanodiamond particle via a hydrophilic polymer chain.
[7] The surface-modified nanodiamond according to any one of [1] to [6], wherein
   the boron cluster-containing group is bonded to a hydrophilic polymer chain via an alkenyl group, a sulfide bond, a sulfonium bond, a sulfoxide group, a secondary amine group, a tertiary amine group, a quaternary amine group (quaternary ammonium cationic group), an amide bond, an ether bond, an ester bond, a thioester bond, a heterocycle (e.g., a 1,2,3-triazole ring), a carbonyl group, an acetal bond, or a group formed by bonding two or more of these groups, and
   the hydrophilic polymer chain is bonded to the nanodiamond particle.
[8] The surface-modified nanodiamond according to any one of [1] to [7], wherein the group capable of recognizing a cancer cell is bonded to the nanodiamond particle via a hydrophilic polymer chain.
[9] The surface-modified nanodiamond according to any one of [1] to [8], wherein the group capable of recognizing a cancer cell is bonded to a hydrophilic polymer chain via an amide bond, an ester bond, or a thioester bond, and
   the hydrophilic polymer chain is bonded to the nanodiamond particle.
[10] The surface-modified nanodiamond according to any one of [1] to [9], wherein
   the group capable of recognizing a cancer cell is a group derived from a molecule capable of recognizing a cancer cell, and
   the molecule capable of recognizing a cancer cell is a boronic acid, an amino acid, a peptide, a protein, folic acid, or a porphyrin.
[11] The surface-modified nanodiamond according to [10], wherein the boronic acid is a compound represented by Formula (A) below:

   R^{a2}-B(-R^{a1})₂ (A)

   (wherein R^{a1} represents a hydroxy group that may be protected by a protective group, and R^{a2} represents a hydrogen atom or a monovalent organic group).
[12] The surface-modified nanodiamond according to [11], wherein the monovalent organic group in the R^{a2} is a substituted or unsubstituted hydrocarbon group (monovalent hydrocarbon group, in particular, a monovalent aliphatic or aromatic hydrocarbon group), a substituted or unsubstituted heterocyclic group (monovalent heterocyclic group), or a group formed by bonding of two or more of the monovalent hydrocarbon group and/or the monovalent heterocyclic group.
[13] The surface-modified nanodiamond according to any one of [10] to [12], wherein the peptide is a peptide having an RGD sequence.
[14] The surface-modified nanodiamond according to any one of [10] to [13], wherein the peptide is at least one selected from the group consisting of GRGDNP (Gly-Arg-Gly-Asp-Asn-Pro), Cyclo(-Arg-Gly-Asp-D-Phe-Cys), Cyclo[-Arg-Gly-Asp-D-Phe-Lys (Cys)], Cyclo(-Arg-Gly-Asp-D-Tyr-Lys), and Cyclo(-Arg-Gly-Asp-D-Phe-Lys).
[15] The surface-modified nanodiamond according to any one of [4] to [14], wherein a number-average degree of polymerization of glycerol in the polyglycerol chain is from 3 to 2000, from 5 to 500, or from 10 to 200.
[16] The surface-modified nanodiamond according to any one of [1] to [15], wherein the boron cluster is at least one selected from the group consisting of a carborane, a dodecaborate, a boron ion cluster having a molecular formula represented by B₁₀H₁₀, and a sandwich-type ion cluster having a molecular formula represented by M(C₂B₉H₁₀)₂ (M is a transition element).
[17] The surface-modified nanodiamond according to any one of [1] to [16], wherein a boron atom in the boron cluster-containing group is enriched in ¹⁰B.
[18] The surface-modified nanodiamond according to any one of [1] to [17], wherein the nanodiamond particle contains a nanodiamond primary particle, or a secondary particle formed by aggregation (adhesion) of a plurality of the primary particles.
[19] The surface-modified nanodiamond according to any one of [1] to [18], wherein the nanodiamond particle is of a detonation nanodiamond.
[20] A composition for use in boron neutron capture therapy, the composition containing the surface-modified nanodiamond described in any one of [1] to [19].

### Industrial Applicability

The surface-modified nanodiamond of the present disclosure enables selective accumulation of a sufficient amount of boron atoms in a cancer tissue or a cancer cell and has high killing and injuring ability against the cancer cell.

## Claims

1. A surface-modified nanodiamond comprising:
a nanodiamond particle; and
a boron cluster-containing group and a group capable of recognizing a cancer cell as surface modification groups of the nanodiamond particle.

2. The surface-modified nanodiamond according to claim 1, further comprising a hydrophilic polymer chain-containing group as a surface modification group of the nanodiamond particle.

3. The surface-modified nanodiamond according to claim 2, wherein a hydrophilic polymer chain in the hydrophilic polymer chain-containing group is a polyglycerol chain.

4. The surface-modified nanodiamond according to claim 1 or 2, wherein the boron cluster-containing group is bonded to the nanodiamond particle via a hydrophilic polymer chain.

5. The surface-modified nanodiamond according to claim 1 or 2, wherein the group capable of recognizing a cancer cell is bonded to the nanodiamond particle via a hydrophilic polymer chain.

6. The surface-modified nanodiamond according to claim 1 or 2, wherein
the group capable of recognizing a cancer cell is bonded to a hydrophilic polymer chain via an amide bond, an ester bond, or a thioester bond, and
the hydrophilic polymer chain is bonded to the nanodiamond particle.

7. The surface-modified nanodiamond according to claim 1 or 2, wherein
the group capable of recognizing a cancer cell is a group derived from a molecule capable of recognizing a cancer cell, and
the molecule capable of recognizing a cancer cell is a boronic acid, an amino acid, a peptide, a protein, folic acid, or a porphyrin.

8. The surface-modified nanodiamond according to claim 1 or 2, wherein a boron atom in the boron cluster-containing group is enriched in ¹⁰B.

9. A composition for use in boron neutron capture therapy, the composition comprising the surface-modified nanodiamond described in claim 1 or 2.
